(19) Europäisches Patentamt
European Patent Office
Office européen des brevets

(11) EP 4 420 678 A1

(12) **EUROPEAN PATENT APPLICATION**
published in accordance with Art. 153(4) EPC

(43) Date of publication:
**28.08.2024 Bulletin 2024/35**

(21) Application number: **22883629.2**

(22) Date of filing: **20.10.2022**

(51) International Patent Classification (IPC):
***A61K 41/00*** (2020.01) ***A61K 31/197*** (2006.01)
***A61K 31/513*** (2006.01) ***A61K 45/00*** (2006.01)
***A61K 47/68*** (2017.01) ***A61N 5/10*** (2006.01)
***A61P 35/00*** (2006.01) ***C12N 5/09*** (2010.01)

(52) Cooperative Patent Classification (CPC):
**A61K 31/197; A61K 31/513; A61K 41/00; A61K 45/00; A61K 47/68; A61N 5/10; A61P 35/00; C12N 5/06**

(86) International application number:
**PCT/JP2022/039079**

(87) International publication number:
**WO 2023/068325 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.10.2021 JP 2021172003**

(71) Applicants:
- **National Institute of Advanced Industrial Science and Technology
Chiyoda-ku
Tokyo 100-8921 (JP)**
- **University of Tsukuba
Tsukuba-shi, Ibaraki 305-8577 (JP)**
- **Taiyo Service Inc.
Hamamatsu-shi, Shizuoka 431-0201 (JP)**

(72) Inventors:
- **IWATA, Yasushi
Tsukuba-shi, Ibaraki 305-8560 (JP)**
- **MATSUI, Hirofumi
Tsukuba-shi, Ibaraki 305-8577 (JP)**
- **SUZUKI, Iwane
Tsukuba-shi, Ibaraki 305-8577 (JP)**
- **SUZUKI, Yuji
Hamamatsu-shi, Shizuoka 431-0201 (JP)**
- **TOMITA, Kanako
Tsukuba-shi, Ibaraki 305-8560 (JP)**
- **YANG, Tianjing
Tsukuba-shi, Ibaraki 305-8577 (JP)**
- **IKEDA, Takafumi
Tsukuba-shi, Ibaraki 305-8577 (JP)**
- **KUROKAWA, Hiromi
Tsukuba-shi, Ibaraki 305-8577 (JP)**

(74) Representative: **Haseltine Lake Kempner LLP
Cheapside House
138 Cheapside
London EC2V 6BJ (GB)**

# (54) ANTICANCER AGENT, PRODRUG OF ANTICANCER AGENT, METHOD FOR KILLING CANCER CELLS IN VITRO, CANCER THERAPY METHOD AND CANCER THERAPY DEVICE

(57) Provided is an anticancer agent containing a substance that contains nitrogen-15 and that specifically accumulates in a cancer cell. Also provided is a method for killing a cancer cell in vitro, including accumulating nitrogen-15 in a cancer cell in vitro and irradiating the cancer cell with a proton beam in vitro. Additionally provided is a cancer treatment method including causing an accumulation of the nitrogen-15 in a cancer cell in a human or a nonhuman animal and irradiating the human or the nonhuman animal with a proton beam.

EP 4 420 678 A1

## Fig. 1

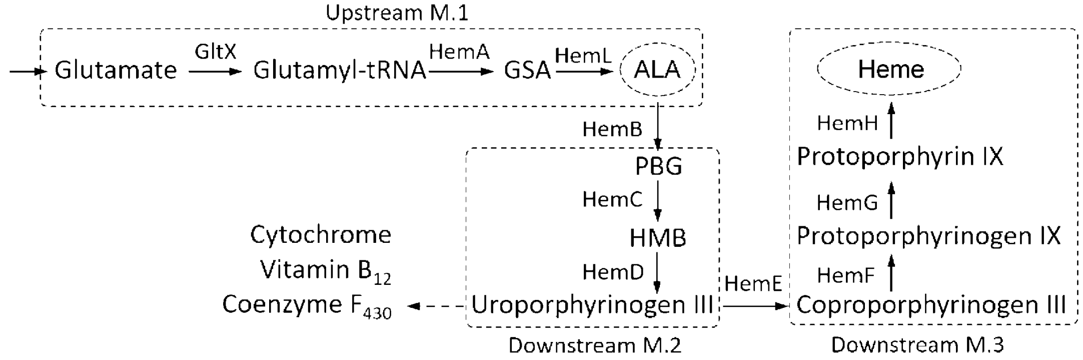
Upstream M.1
Glutamate
GltX
Glutamyl-tRNA
HemA
GSA
HemL
ALA
HemB
PBG
HemC
HMB
HemD
Uroporphyrinogen III
Cytochrome
Vitamin $B_{12}$
Coenzyme $F_{430}$
Downstream M.2
HemE
Coproporphyrinogen III
HemF
Protoporphyrinogen IX
HemG
Protoporphyrin IX
HemH
Heme
Downstream M.3

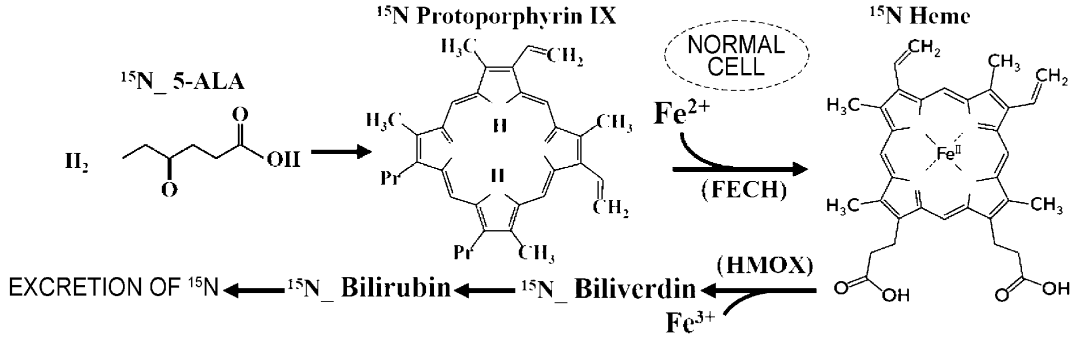
$^{15}N$_ 5-ALA
$^{15}N$ Protoporphyrin IX
$^{15}N$ Heme
NORMAL CELL
$Fe^{2+}$
(FECH)
(HMOX)
$Fe^{3+}$
EXCRETION OF $^{15}N$
$^{15}N$_ Bilirubin
$^{15}N$_ Biliverdin

## Description

### Technical Field

The present invention relates to an anticancer agent, a prodrug of an anticancer agent, a method for killing a cancer cell in vitro, a cancer treatment method, and a cancer treatment device.

### Background Art

Approximately 20,000 individuals are treated annually at proton beam treatment facilities in global, and the total number of patients exceeds 210,000. However, there are 17 million cancer patients globally, and proton beam cancer treatment is thus carried out in a proportion of only 1-in-850 individuals.

### Citation List

#### Patent Documents

Patent Document 1: Japanese Patent No. 4520219

Patent Document 2: Japanese Patent No. 7648586

Patent Document 3: Japanese Patent No. 5392752

#### Non-Patent Documents

Non-Patent Document 1: F. Tabbakh, N. S. Hosmane, Enhancement of Radiation Effectiveness in Proton Therapy: Comparison Between Fusion and Fission Methods and Further Approaches, Scientific Reports 10 (2020) 5466.

Non-Patent Document 2: Toru Tanaka et al., Production of ByProducts During 5-Aminolevulinic Acid Production by a Mutant Photosynthetic Bacteria and Industrial Production of 5-Aminolevulinic Acid Under Temperature Control, Seibutsu Kogaku Kaishi, 93(2015) 24-31.

Non-Patent Document 3: C. ROLFS and W. S. RODNEY, PROTON CAPTURE BY 15N AT STELLAR ENERGIES, Nuclear Physics, A235 (1974) 450-459.

Non-Patent Document 4: G. Imbriani et al., Measurement of $\gamma$ rays from 15N(p,$\gamma$)16O cascade and 15N(p,$\alpha$1$\gamma$)12C reactions, PHYSICAL REVIEW, C85 (2012) 065810.

Non-Patent Document 5: "Torelina (registered trademark) Polyphenylene Sulfide Film" Technical Data, Industrial Materials First Business Unit, Toray Industries, Inc. (2013).

Non-Patent Document 6: M. Tamura, H. Ito, H. Matsui, Radiotherapy for cancer using X-ray fluorescence emitted from iodine, Scientific Reports 7 (2017) 43667.

Non-Patent Document 7: M. Shibuta, et al., Imaging cell picker: A morphology-based automated cell separation system on a photodegradable hydrogel culture platform, J. Bioscience and Bioengineering, 126 (2018) 653-660.

### Summary

#### Technical Problem

According to knowledge held by the present inventors, a wider application of proton beam therapy can be expected if the survival rate of cancer patients treated with proton beam therapy were to be increased. According to knowledge held by the present inventors, proton beam therapy can be expected to raise the survival rate - not only for

heretofore difficult-to-cure organ-specific cancers such as pancreatic cancer, which has a very low 5-year survival rate (8.5%, 2009-2011); lung cancer, which is by far the highest cause of death ranked by individual parts in Japanese men (24.2% of a total of 220,339 cancer deaths in men in 2019); and glioblastoma, which with grade 4 brain tumor symptoms has a low 5-year survival rate of less than 10% - but also for cancer patients with, e.g., lymphomas or dissemination where there is concern that will cause cancer metastasis. Also according to knowledge held by the present inventors, the application of proton beam therapy to breast cancer, for which non-invasive treatments are strongly desired, can be expected.

According to knowledge held by the present inventors, it is desirable to specifically kill cancer cells in order to increase the survival rate of cancer patients and treat cancer efficiently in a non-invasive manner. Therefore, at least a part of the problem to be addressed by the present invention is to provide an anticancer agent that specifically kills a cancer cell, a prodrug of the anticancer agent, a method for killing a cancer cell in vitro, a method for treating cancer, and a device for treating cancer.

Solution to Problem

[1] An anticancer agent that contains a substance that causes a specific accumulation of nitrogen-15 in a cancer cell.

[2] The anticancer agent according to [1], wherein the anticancer agent is to be administered into a body of a patient and the patient is to be irradiated with a proton beam after the administration.

[3] A substance that causes a specific accumulation of nitrogen-15 in a cancer cell, for use in the treatment of cancer.

[4] The substance according to [3], wherein the substance is to be administered into the body of a patient and the patient is to be irradiated with a proton beam after the administration.

[5] The anticancer agent according to [1], wherein the anticancer agent is a molecular-targeted therapeutic drug in which nitrogen is nitrogen-15.

[6] The anticancer agent according to [5], wherein the anticancer agent contains a portion that binds to the cancer cell.

[7] The anticancer agent according to [6], wherein the portion that binds to the cancer cell is an antibody or a part of an antibody.

[8] The anticancer agent according to [7], wherein a drug is bound to the antibody or the part of the antibody.

[9] The anticancer agent according to [7], wherein the antibody or the part of the antibody is trastuzumab in which nitrogen is nitrogen-15.

[10] Use of a substance that causes a specific accumulation of nitrogen-15 in a cancer cell, in manufacturing an anticancer agent.

[11] The use according to [10], wherein the anticancer agent is to be administered into a body of a patient and after administration the patient is to be irradiated with a proton beam.

[12] An anticancer agent containing 5-aminolevulinic acid in which the nitrogen is nitrogen-15.

[13] The anticancer agent according to [12], wherein the anticancer agent is to be administered into the body of a patient and the patient is to be irradiated with a proton beam after the administration.

[14] 5-Aminolevulinic acid in which the nitrogen is nitrogen-15, for use in the treatment of cancer.

[15] The 5-aminolevulinic acid in which the nitrogen is nitrogen-15, according to [14], wherein the 5-aminolevulinic acid in which the nitrogen is nitrogen-15 is to be administered into the body of a patient and the patient is to be irradiated with a proton beam after the administration.

[16] Use of 5-aminolevulinic acid in which the nitrogen is nitrogen-15, in manufacturing an anticancer agent.

[17] The use according to [16], wherein the anticancer agent is to be administered into the body of a patient and after administration the patient is to be irradiated with a proton beam.

[18] An anticancer agent that contains 5-fluorouracil in which a nitrogen is nitrogen-15.

[19] The anticancer agent according to [18], wherein the anticancer agent is to be administered into the body of a patient and the patient is to be irradiated with a proton beam after the administration.

[20] 5-Fluorouracil in which a nitrogen is nitrogen-15, for use in the treatment of cancer.

[21] The 5-fluorouracil in which a nitrogen is nitrogen-15, according to [20], wherein the 5-fluorouracil in which a nitrogen is nitrogen-15 is to be administered into the body of a patient and the patient is to be irradiated with a proton beam after the administration.

[22] Use of 5-fluorouracil in which a nitrogen is nitrogen-15, in manufacturing an anticancer agent.

[23] The use according to [22], wherein the anticancer agent is to be administered into the body of a patient and after administration the patient is to be irradiated with a proton beam.

[24] A prodrug of an anticancer agent that contains a substance that causes a specific accumulation of nitrogen-15 in a cancer cell.

[25] The prodrug according to [24], wherein the prodrug is to be administered into the body of a patient and the patient is to be irradiated with a proton beam after the administration.

[26] A prodrug of an anticancer agent that contains a substance that causes a specific accumulation of nitrogen-15 in a cancer cell, for use in the treatment of cancer.

[27] The prodrug according to [26], which is administered into the body of a patient wherein the patient is irradiated with a proton beam after the administration.

[28] Use of a substance that causes a specific accumulation of nitrogen-15 in a cancer cell, in manufacturing a prodrug of an anticancer agent.

[29] The use according to [28], wherein the prodrug of an anticancer agent is to be administered into the body of a patient and the patient is to be irradiated with a proton beam after the administration.

[30] A prodrug of an anticancer agent that contains 5-fluorouracil in which a nitrogen is nitrogen-15.

[31] The prodrug according to [30], wherein the prodrug is to be administered into the body of a patient and the patient is to be irradiated with a proton beam after the administration.

[32] The prodrug according to [30] or [31], wherein the prodrug is selected from the group consisting of tegafur, tegafur/uracil, tegafur/gimeracil/oteracil potassium, doxifluridine, and capecitabine, in which a nitrogen is nitrogen-15.

[33] A prodrug of an anticancer agent that contains 5-fluorouracil in which a nitrogen is nitrogen-15, for use in the treatment of cancer.

[34] The prodrug according to [33], wherein the prodrug is to be administered into the body of a patient and the patient is to be irradiated with a proton beam after the administration.

[35] The prodrug according to [33] or [34], wherein the prodrug is selected from the group consisting of tegafur, tegafur/uracil, tegafur/gimeracil/oteracil potassium, doxifluridine, and capecitabine, in which a nitrogen is nitrogen-15.

[36] Use of any selection from the group consisting of tegafur, tegafur/uracil, tegafur/gimeracil/oteracil potassium, doxifluridine, and capecitabine, in which a nitrogen is nitrogen-15, in manufacturing a prodrug of an anticancer agent.

[37] The use according to [36], wherein the prodrug of an anticancer agent is to be administered into the body of a

patient and the patient is to be irradiated with a proton beam after the administration.

[38] A method for killing a cancer cell in vitro, which includes (a) causing in vitro an accumulation of nitrogen-15 in a cancer cell; and (b) irradiating the cancer cell with a proton beam in vitro.

[39] The method for killing a cancer cell in vitro according to [38], wherein causing the accumulation of nitrogen-15 in the cancer cell includes administering the anticancer agent according to any of the preceding to the cancer cells.

[40] The method for killing a cancer cell in vitro according to [38], wherein causing the accumulation of nitrogen-15 in the cancer cell includes administering, to the cancer cell, the prodrug of the anticancer agent according to any of the preceding.

[41] A cancer treatment method that includes (a) causing an accumulation of nitrogen-15 in a cancer cell of a human or a nonhuman animal; and (b) irradiating the human or the nonhuman animal with a proton beam.

[42] The cancer treatment method according to [41], wherein causing the accumulation of the nitrogen-15 in the cancer cells of the human or the nonhuman animal includes administering the anticancer agent according to any of the preceding to the human or the nonhuman animal.

[43] The cancer treatment method according to [41], wherein causing the accumulation of the nitrogen-15 in the cancer cells of the human or the nonhuman animal includes administering the prodrug of the anticancer agent according to any of the preceding to the human or the nonhuman animal.

[44] A cancer treatment device containing an irradiator that irradiates a human or a nonhuman animal in which nitrogen-15 is accumulated in a cancer cell with a proton beam.

[45] The cancer treatment device according to [44], wherein the anticancer agent according to any of the preceding has been administered to the human or nonhuman animal.

[46] The cancer treatment device according to [44], wherein the prodrug of the anticancer agent according to any of the preceding has been administered to the human or nonhuman animal.

[47] The cancer treatment device according to any of [44] to [46], which further includes an accelerator that accelerates the proton beam.

[48] The cancer treatment device according to [47], wherein the accelerator includes a laser plasma.

Advantageous Effects of Invention

The present invention can thus provide an anticancer agent that specifically kills a cancer cell, a prodrug of an anticancer agent, a method for killing a cancer cell in vitro, a cancer treatment method, and a cancer treatment device.

Brief Description of Drawings

Fig. 1 is a schematic diagram that shows the pathway by which protoporphyrin IX, derived from 5-aminolevulinic acid, is converted to heme.

Fig. 2 is a schematic diagram that shows the kinetic energy positions due to the $^{15}N(^{1}H, \alpha_1\gamma)^{12}C$ resonance nuclear reaction.

Fig. 3 is a diagram that shows the relationship between the laboratory system and the center-of mass system for the $^{15}N(^{1}H, \alpha_1\gamma)$ $^{12}C$ resonance nuclear reaction.

Fig. 4 shows a comparison of the LETs of the proton and product ions.

Fig. 5 shows the dependence on proton energy of the reaction cross section of the $^{15}N(^{1}H, \alpha_1\gamma)^{12}C$ resonance

nuclear reaction.

Fig. 6 is a diagram that describes the relationship between the resonance energy of the $^{15}N(^1H, \alpha_1\gamma)^{12}C$ resonance nuclear reaction and the reaction position (residual range) of the proton in the body, and describes the range of the reaction product ions on the scale of this residual range.

Fig. 7 shows the resonance energy of the $^{15}N(^1H, \alpha_1\gamma)$ $^{12}C$ resonance nuclear reaction, the reaction position (residual range) of the proton in the body, the range of reaction product ions ($^4He^{2+}$, $^{12}C^{6+}$), and the biological ionization effect (Bragg's peak).

Fig. 8 is a schematic diagram that shows the occurrence of the $^{15}N$ resonance nuclear reaction in cancer cells.

Fig. 9 shows the resistance to thermal hydrolysis of Torelina (registered trademark) (refer to Non-Patent Document 8) .

Fig. 10 is a table that shows the gas permeabilities of Torelina (registered trademark) film (refer to Non-Patent Document 9).

Fig. 11 is a schematic diagram that shows a cancer treatment device according to an embodiment.

Fig. 12 is a diagram that shows a method, according to Example 1, for quantitating the $^{15}N$ that has accumulated in normal cells and in cancer cells.

Fig. 13 is a graph that shows the gamma-ray spectrum obtained in the quantitation according to Example 1 of the $^{15}N$ that has accumulated in normal cells and in cancer cells.

Fig. 14 is a graph that shows the amount of intake of $^{15}N$_5-ALA, according to Example 1, into normal cells and into cancer cells.

Fig. 15 is a gene structural diagram of the pUC4-KIXX plasmid according to Example 2.

Fig. 16 contains photographs that show E. coli colonies when the $^{15}N$ isotopic ratio in pUC4-KIXX was changed according to Example 2.

Fig. 17 is a graph that shows, when the $^{15}N$ isotopic ratio in pUC4-KIXX was changed according to Example 2, the E. coli count (left vertical axis) and the yield of 4.43 MeV gamma rays (right vertical axis) emitted by the $^{15}N(^1H, \alpha_1\gamma)^{12}C$ resonance nuclear reaction ($E_r$ = 0.987 MeV).

Fig. 18 contains photographs of RGM-GFP cells cultured on 15N_5-ALA-free culture medium, immediately after the execution of proton beam irradiation according to Example 3.

Fig. 19 is a graph of the calculated results for the surviving fraction versus the proton beam irradiation charge amount, for RGM-GFP cells cultured on 15N_5-ALA-free culture medium, according to Example 3.

Fig. 20 contains photographs of RGK-KO cells immediately after the execution of proton beam irradiation according to Example 3. These are photographs of cells cultured on 15N_5-ALA-containing culture medium and 15N_5-ALA-free culture medium.

Fig. 21 contains photographs of RGK-KO cells after the elapse of 24 hours after the execution of proton beam irradiation according to Example 3, and contains photographs of cells cultured on 15N_5-ALA-containing culture medium.

Fig. 22 contains a photograph of a cell holder, according to Example 4, that enables cells to be held in a live state in a vacuum.

Fig. 23 is a graph according to Example 4 that shows timewise changes in the vacuum when cells were held in a live state in a vacuum.

Description of Embodiments

Modes for executing the present invention (referred to as "embodiments" in the following) are described below. The present invention is not limited to the following embodiments, and the present invention can be executed in various modifications within the scope of its essential features. The embodiments given in the following are examples of, e.g., methods and so forth, for realizing the technical concepts of this invention and are not limited to these examples.

An anticancer agent according to an embodiment contains a substance that contains nitrogen-15 and that specifically accumulates in a cancer cell.

Nitrogen (N) is one of the six essential abundant elements (O, C, H, N, Ca, P), takes up 2.6% of the weight of a human, and can be present in all regions of the body. Nitrogen-15 is also referred to as $^{15}N$. $^{15}N$ is one of the stable isotopes of naturally occurring nitrogen and is composed of 7 protons and 8 neutrons. $^{15}N$ accounts for 0.364% of the total nitrogen on the earth. $^{15}N$ is also present in the body at the same ratio.

The substance that contains nitrogen-15 and that specifically accumulates in the cancer cell may be 5-Aminolevulinic acid in which the nitrogen is nitrogen-15. 5-Aminolevulinic acid (5-ALA) is synthesized in all cells and is known as a starting material for the porphyrin synthesis pathway. In normal cells, heme, which is necessary for energy metabolism, is synthesized from 5-aminolevulinic acid through seven steps of enzymatic reactions. When released from protein, heme promotes the generation of active oxygen and thus causes oxidative stress that damages DNA and lipids, and it is therefore rapidly degraded and excreted after energy is produced. The chemical formula of 5-aminolevulinic acid is as follows.

[Chemical formula 1]

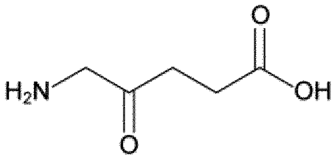

In the anticancer agent according to the embodiment, N in 5-aminolevulinic acid is replaced by $^{15}N$. 5-Aminolevulinic acid in which the nitrogen is nitrogen-15 is also represented by $^{15}N$_5-ALA. The chemical formula of 5-aminolevulinic acid in which the nitrogen is nitrogen-15 is given below.

[Chemical formula 2]

$^{15}N$_5-ALA

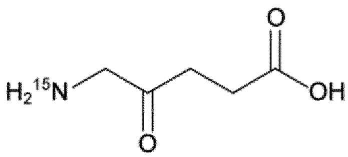

The anticancer agent according to the embodiment is administered to a human or a nonhuman animal. The route of administration can be exemplified by topical administration, enteral administration including oral administration, and parenteral administration, but is not particularly limited. The administered anticancer agent is taken up by cells.

As shown in Fig. 1, protoporphyrin IX derived from 5-aminolevulinic acid in which the nitrogen is nitrogen-15 is converted to heme by ferrochelatase (FECH) in normal cells. In the normal cells, which can normally synthesize the heme, the nitrogen-15 is excreted and does not accumulate.

In cancer cells, however, induced nitric oxide synthase (iNOS) is active and the intracellular nitric oxide (NO) concentration is maintained at high levels. As a consequence, in the cancer cells, the iron-sulfur complex rapidly reacts with nitric oxide (NO) to irreversibly form a dinitrosyldithiolate iron complex (DNIC), and as a result the heme cannot be synthesized. As a consequence, 5-aminolevulinic acid in which the nitrogen is nitrogen-15 and derivatives of 5-aminolevulinic acid in which the nitrogen is nitrogen-15, such as protoporphyrin IX in which the nitrogen is nitrogen-15, accumulate in the cancer cells for a long time.

Thus, when the anticancer agent according to the embodiment is administered to a human or a nonhuman

animal, nitrogen-15 specifically accumulates in cancer cells but does not accumulate in normal cells.

Fig. 2 shows the center-of-mass system energy diagram (unit = MeV) for the proton capture resonance nuclear reaction of the nitrogen-15 atomic nucleus. When the proton and nitrogen-15 collide, in the case where the sum of the binding energy and collision energy for the both atomic nuclei accords (resonates) with the excitation level of the oxygen-16 atomic nucleus, the proton is captured by the nitrogen-15 atomic nucleus to form the oxygen-16 compound atomic nucleus $^{16}O^*$. The $^{16}O^*$, which is in an excited state, emits the $^4He^{2+}$ atomic nucleus ($\alpha$ ray), which is the most stable of the atomic nucleus species, and immediately assumes the first excitation level of the carbon-12 atomic nucleus; a 4.43 MeV gamma ray is emitted from the $^{12}C^*$ excitation level to yield the $^{12}C$ atomic nucleus in the ground state. The formula for the resonance nuclear reaction with this sequence is given by the following formula (1).

$$^{15}N(^1H, \alpha_1\gamma)^{12}C \qquad (1)$$

Here, the 1 in the $\alpha_1$ indicates the first excitation level of $^{12}C^*$, and the energy difference $E_0$ from the $^{16}O^*$ excitation level is distributed according to the law of the conservation of momentum into the kinetic energies of the $^4He$ and $^{12}C^*$. $^4He$ and $^{12}C^*$ are emitted as product ions. When this sequence of reaction processes is viewed as a center-of-mass system, i.e., as a coordinate system that moves at the velocity $V_G$ at which the proton/nitrogen-15 centroid moves, the $^{16}O^*$ compound atomic nucleus is resting. When the mass of the proton and $^{15}N$ and the velocity in the laboratory system are made, respectively, $m_H$, $m_N$, $u_H$, and $u_N = 0$, the center-of-mass velocity is given by the following formula (2).

[Math. 1]

$$V_G = \frac{m_H}{m_H+m_N} u_H + \frac{m_N}{m_H+m_N} u_N = \frac{m_H}{m_H+m_N} u_H. \qquad (2)$$

Fig. 3 shows the relationship between the laboratory system and the center-of-mass system. The velocities $V_H$ and $V_N$ of the proton and $^{15}N$ in the center-of-mass system are given by $u_H - V_G$ and $- V_G$, respectively, and the resonance energy $\varepsilon_0$ is equal to the kinetic energy of the proton in the laboratory system, as shown in the following formula (3).

[Math. 2]

$$\varepsilon_0 = \frac{m_H V_H{}^2}{2} + \frac{m_N V_N{}^2}{2} = \frac{m_H u_H{}^2}{2}. \qquad (3)$$

The energy difference between the resonance energy level of the $^{16}O^*$ compound atomic nucleus and the first excitation level of $^{12}C^*$ conserves the momenta of the product ions, that is, is distributed into the kinetic energies of the product ions such that the vector sum of the momenta of the respective product ions becomes equal to the zero momentum of the at-rest $^{16}O^*$ compound atomic nucleus ($m_{He}V_{He} + m_cV_c = 0$) .

[Math. 3]

$$E_0 = \frac{m_{He} V_{He}{}^2}{2} + \frac{m_C V_C{}^2}{2} = \varepsilon_{He} + \varepsilon_C. \qquad (4)$$

The maximum values of the kinetic energies $\varepsilon_{He}$ and $\varepsilon_C$ of $^4He$ and $^{12}C^*$ in the laboratory system are given by the following formula (5) and formula (6).

[Math. 4]

$$\varepsilon_{He} = \frac{1}{2} m_{He}(V_{He} \pm V_G)^2 = \frac{m_{He}}{m_{He}+m_C} E_0 + \frac{m_H m_C}{(m_H+m_N)^2} \varepsilon_0 \pm \sqrt{\frac{m_C}{m_{He}+m_C} \frac{m_H m_{He}}{(m_H+m_N)^2} E_0 \varepsilon_0}. \qquad (5)$$

[Math. 5]

$$\varepsilon_C = \frac{1}{2} m_C(V_C \pm V_G)^2 = \frac{m_{He}}{m_{He}+m_C} E_0 + \frac{m_H m_C}{(m_H+m_N)^2} \varepsilon_0 \pm \sqrt{\frac{m_{He}}{m_{He}+m_C} \frac{m_H m_C}{(m_H+m_N)^2} E_0 \varepsilon_0}. \qquad (6)$$

The kinetic energies $\varepsilon_{He}$ and $\varepsilon_C$ given by the preceding formulae (5) and (6) determine the energy transferred from the ions to the surroundings along the track of the product ions (energy-transferring linear energy transfer (LET) per unit range) and define the track of the product ions. Since LET is proportional to the square of the charge on the ion, $Z^2$, when a $^4He^{2+}$ atomic nucleus having Z = 2 and a $^{12}C^{6+}$ atomic nucleus having Z = 6 are emitted within the cell as product ions, the $^4He^{2+}$ atomic nucleus and $^{12}C^{6+}$ atomic nucleus travel within the cell at high kinetic energies and impart high energies to the surroundings and thus can have a cell-killing capability. Fig. 4 is a graph showing a comparison of the LET of the proton and product ions ($^4He^{2+}$ atomic nucleus and $^{12}C^{6+}$ atomic nucleus). The product ions exhibit a higher LET than the proton, and, with cancer cells in which $^{15}N$ has accumulated, it becomes possible to specifically kill the cancer cells with the product ions from the $^{15}N$ resonance nuclear reaction.

In addition to the reaction channel of formula (1), the following can also proceed from the excited nucleus level of the $^{16}O^*$ compound atomic nucleus: a direct de-excitation reaction to the ground state of carbon, and a reaction in which only $\gamma$ radiation is emitted, without $\alpha$ emission, to achieve the ground state of oxygen-16. When these are expressed with formulae, the reaction channels of the following formula (7) and formula (8) are simultaneously open, respectively.

$$^{15}N(^1H, \alpha_0)^{12}C \qquad (7)$$

$$^{15}N(^1H, \gamma_0)\ ^{16}O \qquad (8)$$

In the resonance nuclear reaction given by formula (8), only highly penetrating gamma rays are emitted and the cancer cell-specific radiation action that is the goal of the present embodiment is not brought about. However, due to the extremely high stability of the $^4He^{2+}$ atomic nucleus and the $^{12}C^*$ atomic nucleus, the resonance nuclear reaction channels represented by formula (1) and formula (7) are produced at overwhelmingly higher probabilities than the resonance nuclear reaction channel represented by formula (8). The order of nuclear reaction probabilities is given by the following formula (9).

(reaction probability of formula 1) > (reaction probability of formula 7) >> (reaction probability of formula 8)

As, in the cancer cells that have accumulated a large amount of nitrogen-15, a resonance nuclear reaction in which a proton collides with the nitrogen-15 atomic nucleus and product ions are emitted occurs with high probability, it is possible to specifically kill the cancer cells.

Fig. 5 shows the reaction cross section for each proton resonance energy, specified by formula (3), of the $^{15}N(^1H, \alpha_1\gamma)^{12}C$ resonance nuclear reaction, and corresponding to the excitation level of the $^{16}O^*$ compound nucleus (refer to Non-Patent Documents 3 and 4). Fig. 6 shows the residual range (residual range from the p-stopping point), which shows the reaction position of the proton in the body for the resonance energy of the proton beam, and shows the range of the reaction product ions on the scale of this residual range. 12 resonance energy positions exist in the range where the proton enters the body and is attenuated to an energy of 10 MeV or less, in a region of about 800 um as the residual range. At the residual range positions corresponding to each resonance energy, the $^4He^{2+}$ atomic nucleus and $^{12}C^{6+}$ atomic nucleus product ions are emitted at the kinetic energies given by formula (5) and formula (6), and the range of each product ion is indicated by the radius of the small circle at each resonance energy. High energy is applied to the surroundings over this product ion range and the surroundings are excited at high densities. Damage is thus produced in the cancer cells at these 12 resonance energy positions and the cancer cells die. Fig. 7 gives the resonance energy of the $^{15}N(^1H, \alpha_1\gamma)^{12}C$ resonance nuclear reaction, the reaction position (residual range) of protons in the body, the range of reaction product ions ($^4He^{2+}$, $^{12}C^{6+}$), and the biological ionization effect (Bragg's peak).

In the cancer cells to which the anticancer agent according to the embodiment has been provided, particularly at the mitochondria protoporphyrin IX in which the nitrogen is nitrogen-15 is synthesized in large amounts. Due to this, and while not wishing to be bound by theory, it is believed that, in the cancer cells and as shown in Fig. 8, the 15N resonance nuclear reaction occurs at high levels upon proton beam irradiation, the mitochondrial are then damaged, and the cancer cells are killed.

The cancer cells in the body of a human or nonhuman animal can thus be specifically killed by the proton beam irradiation of the human or nonhuman animal to whom the anticancer agent according to the embodiment has been administered.

The method for manufacturing 5-aminolevulinic acid in which the nitrogen is nitrogen-15 is not particularly limited. 5-Aminolevulinic acid is ordinarily biosynthesized by supplying glycine and succinic acid, the precursors for 5-aminolevulinic acid, to a 5th mutant strain (CR-520), 6th mutant strain (CR-606), or 7th mutant strain (CR-720) capable of producing 5-aminolevulinic acid under aerobic and dark conditions and created on the basis of the photosynthetic bacterium Rhodobacter sphaeroides IFO12203.

Therefore, it is possible to manufacture 5-Aminolevulinic acid in which the nitrogen is nitrogen-15 by supplying

glycine in which the nitrogen is nitrogen-15 and succinic acid in which the nitrogen is nitrogen-15 to bacteria that produce 5-aminolevulinic acid.

When 5-aminolevulinic acid in which the nitrogen is nitrogen-15 is manufactured by biosynthesis, a large amount of nitrogen is consumed in the processes of synthesizing, e.g., proteins, nucleic acids, and so forth, in addition to the metabolic pathway for 5-ALA, and it is desirable to efficiently utilize the nitrogen-15, which is rare and exists at only 0.364% in nature. Due to this, it is desirable to recover the $^{15}N$ that is contained in large amounts in the residue after the biosynthetic production of 5-aminolevulinic acid in which the nitrogen is nitrogen-15, and to return this to the 5-aminolevulinic acid production process. An example of a method for recovering the nitrogen from the residue is the Kjeldahl method, in which organic nitrogen is heated in the presence of sulfuric acid and recovered as the $(NH_4)^+$ ion. Nitrogen utilization in, e.g., microalgae, *Escherichia coli,* etc., supports the direct uptake of the $(NH_4)^+$ ion and is suitable as a recovery route for nitrogen-15. The intaken $(NH_4)^+$ ion is taken into the glutamate synthesis cycle by glutamine synthetase with the synthesis of glutamic acid. The C5 pathway - in which 5-ALA is synthesized by three enzymes (GltX, HemA, and HemL) from glutamic acid synthesized via $\alpha$-ketoglutarate ($\alpha$-KG) in the glucose-utilizing tricarboxylic acid (TCA) cycle - can be used as a means for producing 5-aminolevulinic acid in which the nitrogen is nitrogen-15 as a biopharmaceutical.

The nitrogen-15-containing substance that specifically accumulates in the cancer cells is not limited to 5-aminolevulinic acid in which the nitrogen is nitrogen-15. For example, the nitrogen-15-containing substance that specifically accumulates in the cancer cells may be 5-fluorouracil in which a nitrogen is nitrogen-15. Or, the nitrogen-15-containing substance that specifically accumulates in the cancer cells may be a prodrug of 5-fluorouracil in which a nitrogen is nitrogen-15. The prodrug of 5-fluorouracil in which a nitrogen is nitrogen-15 can be exemplified by tegafur in which a nitrogen is nitrogen-15, tegafur/uracil in which a nitrogen is nitrogen-15, tegafur/gimeracil/oteracil potassium in which a nitrogen is nitrogen-15, doxifluridine in which a nitrogen is nitrogen-15, and capecitabine in which a nitrogen is nitrogen-15.

In addition, the nitrogen-15-containing substance that specifically accumulates in cancer cells according to the embodiment may be a molecular-targeted therapeutic drug in which a nitrogen is nitrogen-15. Molecular-targeted therapeutic drugs, for example, have a portion that binds to cancer cells. Molecular-targeted therapeutic drugs, for example, have a portion that binds to a biomolecule that is specifically expressed by cancer cells. For example, a portion that binds to a biomolecule that is specifically expressed by cancer cells may contain nitrogen-15. The portion that binds to a biomolecule that is specifically expressed by cancer cells may be an antibody or a part of an antibody. The molecular-targeted drug may be an antibody-drug conjugate (ADC) that has nitrogen-15 for its nitrogen and that is targeted to and kills tumor cells while sparing healthy cells. The molecular-targeted drug may be an antibody or part of an antibody that has nitrogen-15 for its nitrogen and that targets the HER2 (human epidermal growth factor receptor 2) glycoprotein present at the surface of breast cancer cells. A HER2-targeting antibody or part of an antibody in which a nitrogen is nitrogen-15 may be obtained, for example, by substituting nitrogen-15 for the nitrogen in trastuzumab (trade name: Herceptin).

The $^{15}N$ used in the cancer treatment drug according to the embodiment is one of the six essential abundant elements and can be supplied to all regions of the body.

The $^{15}N$ used in the cancer treatment drug according to the embodiment accounts for only 0.364% of the total nitrogen in nature, and it is possible to specifically kill the cancer cells by the accumulating the $^{15}N$ anticancer agent in high proportions in the cancer cells.

The $^{15}N$ used in the cancer treatment drug according to the embodiment is a stable isotopic element. When the N in an already approved cancer treatment drug is replaced with $^{15}N$ and the cancer treatment drug having N replaced with $^{15}N$ is administered to a cancer patient, the burden on the patient will be equivalent to that for the already approved cancer treatment drug.

The chemical action of the $^{15}N$ used in the cancer treatment drug according to the embodiment is entirely the same as that of nitrogen-14 ($^{14}N$), which exists in nature at 99.636%. In the case where an already approved cancer treatment drug contains nitrogen, 99.636% of the nitrogen is $^{14}N$. Even if the $^{14}N$ is replaced with $^{15}N$ at high concentrations, for example, at a ratio of 98% or more, the chemical action of the cancer treatment drug in the body is entirely the same as the already approved cancer treatment drug and the toxicity does not change.

A method for killing a cancer cell in vitro according to the embodiment shows the therapeutic effect of the nitrogen-15-containing cancer treatment drug. Since the method for killing a cancer cell in vitro according to the embodiment makes it possible to irradiate the cancer cell with the proton beam while controlling the irradiation energy of the proton beam to the resonance energy at which the $^{15}N$ resonance nuclear reaction occurs, it is possible to accurately evaluate the therapeutic effects of the nitrogen-15-containing cancer treatment drug.

This method for killing a cancer cell in vitro includes sealing a cancer cell and optional normal cell, along with the culture solution, in a vacuum using a polymer film. By doing this, the cells in the vacuum chamber through which the proton beam passes can be maintained in a living state.

The polymer film that seals the cancer cell and optional normal cell along with the culture solution in a vacuum

in the method for killing a cancer cell in vitro, may be a polyethylene sulfide material, and a film commercially available as Torelina (Toray Industries, Inc.) may be used. As shown in Fig. 9, polyethylene sulfide film is very stable to thermal hydrolysis (refer to Non-Patent Document 5). In addition, a property of Torelina, as shown in Fig. 10, is a high permeability for oxygen, nitrogen, and carbon dioxide, but a very low permeability for water vapor (refer to Non-Patent Document 5).

Even in an environment of exposure to a high-density excitation action due to the proton beam while in contact with an aqueous solution for the culture, a stable sealing of the cancer cells and optional normal cells along with the culture solution in a vacuum is made possible utilizing the extremely stable properties of Torelina film with respect to thermal hydrolysis. Due to the property of Torelina film of a very low water vapor permeability and due to the very high hydrophobicity of Torelina fiber at the interface where the aqueous solution and Torelina film are in contact, even when the vacuum side surface of the film assumes a negative pressure, it is difficult for the water molecules to become water vapor and the culture solution can be present in a liquid state. In the interval in which the culture solution maintains the liquid state, the highly permeable oxygen and carbon dioxide are maintained in a dissolved state in the liquid and an environment is obtained in which the oxygen and carbon dioxide required to maintain cells in a living state are maintained in the culture solution.

In order to confine the cancer cells and optional normal cells along with the culture solution in a vacuum, a device may be used that has the capability of sealing with a polymer film such as Torelina film. This sealing-capable device may be configured such that the vacuum required for the irradiation of a high energy proton beam is obtained, desirably a vacuum of $1 \times 10^{-3}$ Pa or below. This sealing-capable device may have CLEANSTAR B (product name, Daido Steel Co., Ltd.), a soft stainless steel with an ultralow carbon content (equal to or less than 0.007%), for a material. This sealing-capable device may have a sealing surface having a semicircular cross section. The configuration may be such that a high hardness layer of iron nitride is formed in a surface layer thickness of 100 nm by $N_2$ ion implantation at this sealing surface, to have close contact by elastic deformation of the sealing surface upon tightening and have an easy separation behavior when opening (refer to Patent Documents 2 and 3).

The nitrogen-15-containing anticancer agent is administered to the cancer cells, and optionally to the normal cells, prior to the introduction of the cancer cells and optional normal cells into the vacuum chamber. Sealing with the polymer film, e.g., Torelina film, at the sealing surface is carried out and the cancer cells and optional normal cells are maintained along with the culture solution in a live state in the vacuum chamber. As a consequence of this, the cancer cells and optional normal cells, which have taken up $^{15}N$ into the cell along with the anticancer agent, are maintained in the vacuum chamber. Accordingly, the cancer cells can be killed in vitro by producing the resonance nuclear reaction through the collision of protons with the $^{15}N$ within the cancer cells and optional normal cells.

The cancer treatment device according to the embodiment comprises, as shown in Fig. 11, an irradiator 20 that irradiates a proton beam onto a human 10 or a nonhuman animal in whom nitrogen-15 has accumulated in cancer cells. The above-described anticancer agent or anticancer agent prodrug has been administered to the human 10 or nonhuman animal. The cancer treatment device according to the embodiment may additionally comprise an accelerator that accelerates the proton beam. The accelerator may comprise a laser plasma.

(Example 1)

Rat gastric mucosal-derived cells (RGM-GFP) were prepared as normal cells and rat gastric mucosal-derived cancer cells (RGK-KO) were prepared as cancer cells, and the difference in cellular intake of $^{15}N$_5-ALA between the RGM-GFP cells and RGK-KO cells was confirmed as follows. The rat gastric mucosal-derived cells and rat gastric mucosal-derived cancer cells are both derived from the rat and are widely used in research on anticancer agents because they have the same gene sequence (refer to Non-Patent Documents 6 and 7).

The RGM-GFP cells and RGK-KO cells were cultured for 3 or 4 days on a culture solution adapted to each and were grown to 80% confluence; this was followed by the addition of $^{15}N$_5-ALA and the amount of $^{15}N$ intake per cell with elapsed time was measured. A schematic diagram of measurement of the amount of $^{15}N$ intake is given in Fig. 12. $^{15}N$_5-ALA was added to the RGM-GFP cells and RGK-KO cells that had grown to 80% confluence, and cell samples were prepared after the passage of each of 0 hours, 0.5 hours, 1 hour, 3 hours, 6 hours, 12 hours, and 24 hours after the addition. Washing with fresh culture solution was performed three times in order to remove the $^{15}N$_5-ALA that remained on the cell surface. The cells, which had grown with attachment to the bottom of the Petri dish, were detached from the Petri dish by the addition of trypsin, and the concentration of the liberated cells was measured.

The concentration was adjusted, and $10^5$ cells/2 $\mu$L was dripped onto a crystalline silicon substrate and drying was carried out. The sample size was adjusted to a diameter of approximately 2.5 mm. The cell sample, set into a sample holder, was inserted into a vacuum chamber and was exposed to a proton beam. Considering the thickness of the sample and considering that the energy of the proton beam attenuates in the interior of the sample, the energy of the proton beam was set at 22 points between 0.894 MeV and 0.994 MeV so the $^{15}N$ in the sample interior could be quantitated by the $^{15}N(^1H, \alpha_1\gamma)^{12}C$ resonance nuclear reaction ($E_r$ = 0.897 MeV). The 4.43 MeV gamma radiation emitted by this resonance nuclear reaction with $^{15}N$ was measured using a $Bi_4Ge_3O_{12}$ (BGO) detector.

The measured gamma-ray spectra are given in Fig. 13. Since the 4.43 MeV main peak and the two annihilation gamma ray peaks (3.92 MeV S.E. and 3.41 MeV D.E.) formed a broad peak, the gamma ray yield in the 2.98 MeV to 4.85 MeV energy range was integrated and the natural background (white dots in the figure) was subtracted to provide the effective gamma-ray yield. The cellular intake of $^{15}N$_5-ALA was determined as the absolute value based on the gamma ray yield from samples provided by dripping an aqueous solution of $^{15}N$_5-ALA of stipulated concentration. The results are given in Fig. 14. At 24 hours after the addition of the $^{15}N$_5-ALA, the intake of $^{15}N$_5-ALA by cancer cells was at least 5-times the intake of $^{15}N$_5-ALA by the normal cells.

On the basis of these results, it was shown that the nitrogen-15 taken in as $^{15}N$_5-ALA by the cancer cells accumulated within the cells and the cancer cells could be specifically killed.

The specific procedure for quantitating the $^{15}N$_5-ALA intake by the RGM-GFP cells and RGK-KO cells was as follows.

RGM-GFP and RGK-KO were cultured in 35-mm dishes in an incubator (37°C, 5% $CO_2$) over 3 to 4 days until 80% confluence.

1000 μL of a culture medium solution containing 1 mmol/L $^{15}N$_5-ALA was introduced into each dish. Treatment with trypsin was performed on each of the cells at each of the stipulated elapsed times.

Washing of the cells with phosphate-buffered saline (PBS) or 5% glucose solution and centrifugal separation was carried out three times.

The cell count was measured using an automatic counter (Invitrogen Countess (registered trademark)) and was adjusted to $1.0 \times 10^5$ cell/2 μL. The cells were dripped onto a silicon substrate and after drying the silicon substrate was then set in a vacuum chamber. The cells on the silicon substrate were irradiated with a proton beam from a 1 MV tandem accelerator (Applied Accelerator Department, Tsukuba University), and the 4.43 MeV gamma rays emitted by the resonance nuclear reaction with $^{15}N$ were measured using a BGO detector located outside of the vacuum chamber.

The proton beam was irradiated onto a sapphire plate and the beam area was measured from the fluorescence image, and the gamma ray yield normalized to the unit proton beam charge amount was determined from the relative ratio with the area of the sample dripped onto the silicon substrate.

The intracellular nitrogen-15 was quantitated by normalizing the gamma ray yield from the aqueous $^{15}N$_5-ALA solution sample of stipulated concentration to the proton beam charge amount and comparing the proton beam charge amount-normalized gamma ray yield.

The following reagents were used in Example 1.

5-aminolevulinic acid hydrochloride: 018-13133 (FUJIFILM Wako Pure Chemical Corporation)

$^{15}N$-aminolevulinic acid hydrochloride: N1371 (Medical Isotope)

0.5% Trypsin-EDTA (10X): 15400054

TrypLE Select Enzyme (1X), no phenol red 100 mL: 12563011 (Gibco)
10×D-PBS: 048-29805 (FUJIFILM Wako Pure Chemical Corporation)

D(+)-glucose: 049-31165 (FUJIFILM Wako Pure Chemical Corporation)

(Example 2)

Fig. 15 shows the gene structure of pUC4-KIXX (3914 bps), which is circular artificial plasmid DNA. Because pUC4-KIXX has an ampicillin-resistance gene and a kanamycin-resistance gene, E. coli transformed with pUC4-KIXX exhibits resistance to ampicillin and kanamycin. Modified pUC4-KIXX's were prepared by changing the isotopic ratio $^{15}N/(^{14}N + ^{15}N)$ for the N in pUC4-KIXX in steps to the natural abundance ratio of 0.364% and to 25%, 50%, 75%, and at least 98%.

E. coli transformed with each of the modified pUC4-KIXX's was cultured on culture medium containing 100 ug/mL ampicillin and culture medium containing 20 ug/mL kanamycin, and was subjected to proton beam irradiation. According to the results, and as shown in Fig. 16 and Fig. 17, in comparison to E. coli transformed with pUC4-KIXX not containing $^{15}N$, E. coli transformed with $^{15}N$-containing pUC4-KIXX presented an increase in the number of cells killed by proton beam irradiation as the $^{15}N$ isotopic ratio increased. In comparison to E. coli transformed with pUC4-KIXX not containing $^{15}N$, the colony count for E. coli transformed with pUC4-KIXX having a $^{15}N$ isotope abundance ratio of 75% or more was decreased by at least 1 over $10^3$.

Proton beam irradiation did not kill large numbers of the E. coli transformed with pUC4-KIXX not containing $^{15}N$. It is thus thought that the killing of large numbers of E. coli transformed with a modified pUC4-KIXX was due to a loss of drug resistance by the E. coli due to damage to the ampicillin-resistance gene and kanamycin-resistance gene

due to the $^{15}N(^{1}H, \alpha_1\gamma)^{12}C$ resonance nuclear reaction.

(Example 3)

Rat gastric mucosal-derived cancer cells (RGK-KO) and normal cells (RGM-GFP) were uniformly cultured for seven days in 35-mm dishes filled with culture medium adapted to each, and the cells were prepared so all the dishes reached 80% confluence. $^{15}$N_5-ALA was introduced into the culture medium at 24 hours before proton beam irradiation, and holding was carried out for 24 hours using the same culture conditions for both culture media in order to enable comparison with the culture medium that did not contain $^{15}$N_5-ALA. The RGK-KO cells and RGM-GFP cells cultured on a culture medium containing $^{15}$N_5-ALA and the RGK-KO cells and RGM-GFP cells cultured on a culture medium not containing $^{15}$N_5-ALA were in each instance held sealed in a vacuum along with the culture medium and were irradiated with a proton beam at a prescribed ion charge amount. With regard to the proton beam energy, considering the attenuation of the proton beam energy by the Torelina film and the culture solution sealed in the vacuum, the sample was irradiated at 1.3 MeV to match the 1.21 MeV resonance energy.

Fig. 18 shows fluorescence microscope photographs taken immediately after (within 3 hours) the proton beam irradiation of RGM-GFP cells cultured on culture medium that did not contain $^{15}$N_5-ALA. The upper sections show images of RGM-GFP cells made to metabolize a fluorescent protein by genetic recombination; the lower sections each show photographs in which the dead cells are identified by the DAPI staining method, which stains the DNA in the nucleus of dead cells. The cell mortality, i.e., the number of fluorescent protein-imaged cells (number of surviving cells) versus the sum of the number of fluorescent protein-imaged cells and the number of DAPI staining fluorescent-imaged cells (number of dead cells), increased accompanying the increase in the irradiation charge amount of the proton beam to 2.5 nC, 4.0 nC, 5.0 nC, 10 nC, and 25 nC. Fig. 19 shows the relationship between the irradiated charge amount and the cell surviving fraction for the proton beam irradiation of RGM-GFP cells cultured on culture medium that did not contain $^{15}$N_5-ALA. A logarithmic relationship is seen between the two, and the characteristics of the biological radiation damage due to a proton beam were demonstrated.

Fig. 20 shows photographs for immediately after the proton beam irradiation of RGK-KO cells cultured on culture medium that did not contain $^{15}$N_5-ALA and culture medium that contained $^{15}$N_5-ALA. The photographs in Fig. 20 show the case of a proton beam irradiation amount of charge of 4.0 nC for both cells. Genes for the metabolism of fluorescent protein are also incorporated in the RGK-KO cells, and the cells present a red color when observed with a fluorescence microscope. For the RGK-KO cells cultured on culture medium that did not contain $^{15}$N_5-ALA, the number of cells in the fluorescent protein image was 4671 and the number of cells in the DAPI staining fluorescent image was 3179, and the surviving fraction was 59.5%. On the other hand, for the RGK-KO cells cultured on culture medium that contained $^{15}$N_5-ALA, the number of cells in the fluorescent protein image was 4522 and the number of cells in the DAPI staining fluorescent image was 1622, and the surviving fraction was 73.5%.

Fig. 21 gives photographs of the cells after the passage of 24 hours after proton beam irradiation, at a charge amount of 4.0 nC, for RGK-KO cells that had been cultured on culture medium that contained $^{15}$N_5-ALA. For the cells that exhibited a surviving fraction within three hours immediately after irradiation of 73.5% and 85.5%, the surviving fraction after 24 hours had declined to 40.5% and 10.6%, respectively. The trend of the RGK-KO cell surviving fraction declining sharply with elapsed time shows that the $^{15}$N_5-ALA taken up by the RGK-KO cells is connected to the metabolic pathway in the cancer cells that leads to protoporphyrin IX.

(Example 4)

In a method for killing cancer cells in vitro, the RGM-GFP cells and RGK-KO cells were sealed using a vacuum sealing joint (referred to as a "CS flange" in the following) having a sealing capability, for which the materials were Torelina film (thickness of 4 $\mu$m, Toray Industries, Inc.) and CLEANSTAR; and this was set in a vacuum chamber and held in a vacuum. Fig. 22 shows a photograph in which a crystalline silicon substrate with a diameter of 15 mm and a thickness of 0.5 mm has been placed in a 1.0 mm-deep circular dish made of high-purity silicon; the RGM-GFP cells and RGK-KO cells with culture solution have been dripped onto this substrate; and sealing with Torelina film has been carried out. It was possible to avoid discharge damage due to charging from the proton beam by carrying out the vapor deposition (Toray KP Films Inc.) of a thin carbon film on the surface on one side of the Torelina film.

When RGM-GFP cells and RGK-KO cells that had been kept in a vacuum for 20 to 30 minutes were released from the seal and cultured, regrowth of the cells was confirmed for both cell types, as shown in the cell photograph in Fig. 22, and maintenance in a living condition in a vacuum for a certain period of time was demonstrated.

According to Fig. 23, when RGM-GFP cells and RGK-KO cells were sealed together with the culture solution and vacuum evacuation was performed using a dry pump (TSU071E, exhaust speed = 60 L/s, achieved vacuum = $10^{-5}$ Pa, PFEIFFER), it was confirmed that the vacuum dropped at a rate that was unchanged from the vacuum exhaust process when a cell sample was not present.

## Claims

1. An anticancer agent comprising a substance that causes a specific accumulation of nitrogen-15 in a cancer cell.

2. The anticancer agent according to claim 1, wherein the anticancer agent is to be administered into a body of a patient and the patient is to be irradiated with a proton beam after the administration.

3. The anticancer agent according to claim 1, wherein the substance is 5-aminolevulinic acid in which nitrogen is nitrogen-15.

4. The anticancer agent according to claim 1, wherein the substance is 5-fluorouracil in which nitrogen is nitrogen-15.

5. The anticancer agent according to claim 1, wherein the anticancer agent is a molecular-targeted therapeutic drug in which nitrogen is nitrogen-15.

6. The anticancer agent according to claim 5, comprising a portion that binds to the cancer cell.

7. The anticancer agent according to claim 6, wherein the portion that binds to the cancer cell is an antibody or a part of an antibody.

8. The anticancer agent according to claim 7, wherein a drug is bound to the antibody or the part of the antibody.

9. The anticancer agent according to claim 7, wherein the antibody or the part of the antibody is trastuzumab in which nitrogen is nitrogen-15.

10. A prodrug of an anticancer agent comprising a substance that causes a specific accumulation of nitrogen-15 in a cancer cell.

11. The prodrug according to claim 10, wherein the prodrug is to be administered into a body of a patient and the patient is to be irradiated with a proton beam after the administration.

12. The prodrug according to claim 10, wherein the anticancer agent is 5-fluorouracil in which nitrogen is nitrogen-15.

13. The prodrug according to claim 10, wherein the prodrug is selected from the group consisting of tegafur, tegafur/uracil, tegafur/gimeracil/oteracil potassium, doxifluridine, and capecitabine, in which nitrogen is nitrogen-15.

14. A method for killing a cancer cell in vitro, comprising: causing in vitro an accumulation of nitrogen-15 in a cancer cell; and irradiating the cancer cell with a proton beam in vitro.

15. The method for killing a cancer cell in vitro according to claim 14, wherein causing the accumulation of the nitrogen-15 in the cancer cell comprises administering the anticancer agent according to claim 1 to the cancer cell.

16. The method for killing a cancer cell in vitro according to claim 14, wherein causing the accumulation of the nitrogen-15 in the cancer cell comprises administering, to the cancer cell, a prodrug of the anticancer agent according to claim 5.

17. A cancer treatment method comprising: causing an accumulation of nitrogen-15 in a cancer cell of a human or nonhuman animal; and irradiating the human or nonhuman animal with a proton beam.

18. The cancer treatment method according to claim 17, wherein causing the accumulation of the nitrogen-15 in the cancer cell of the human or the nonhuman animal comprises administering the anticancer agent according to claim 1 to the human or the nonhuman animal.

19. The cancer treatment method according to claim 17, wherein causing the accumulation of the nitrogen-15 in the cancer cell of the human or the nonhuman animal comprises administering a prodrug of the anticancer agent according to claim 5 to the human or the nonhuman animal.

20. A cancer treatment device comprising an irradiator that irradiates a human or a nonhuman animal in which nitrogen-15 is accumulated in a cancer cell with a proton beam.

**21.** The cancer treatment device according to claim 20, further comprising an accelerator that accelerates the proton beam.

**22.** The cancer treatment device according to claim 21, wherein the accelerator comprises a laser plasma.

Fig. 1

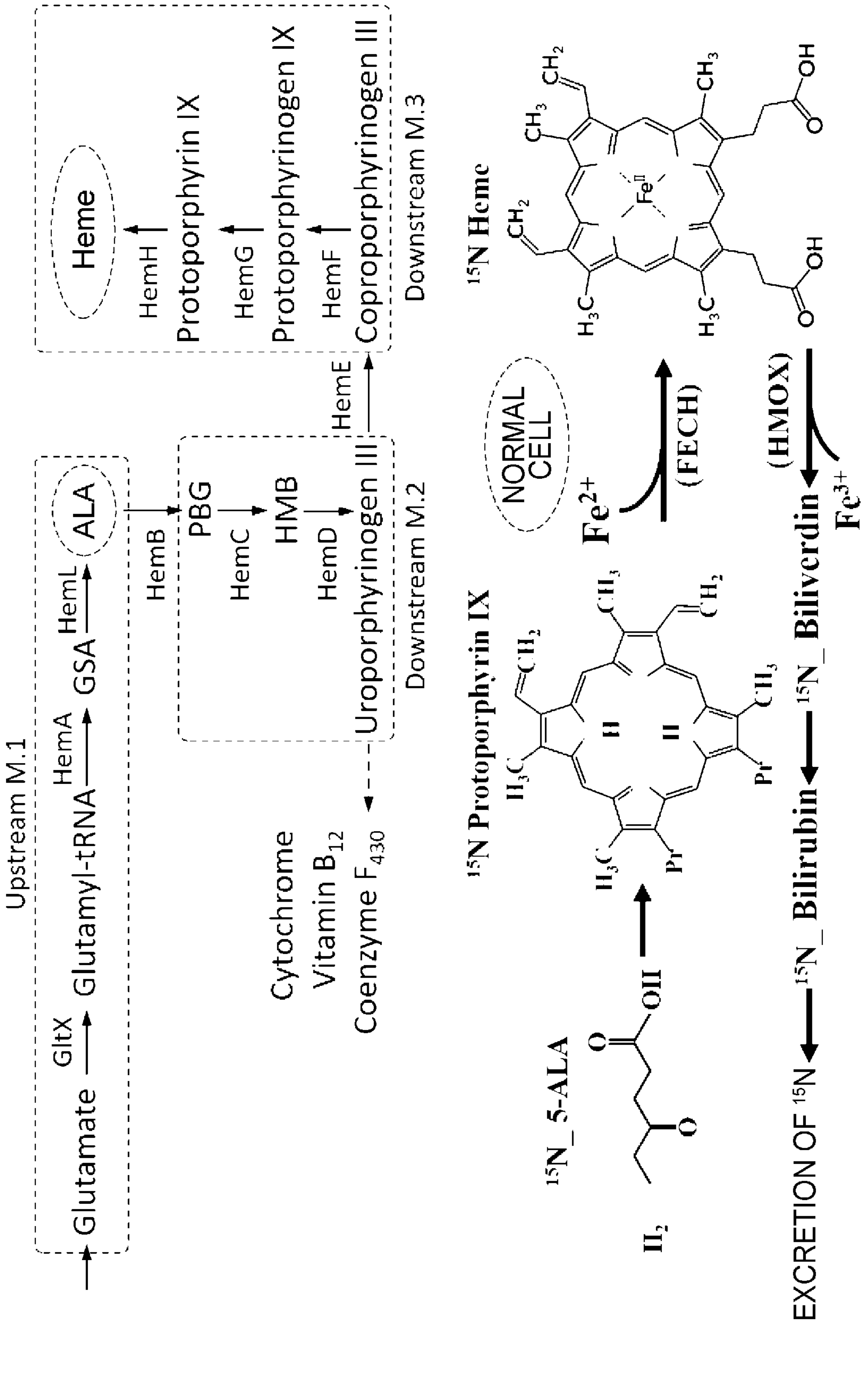
Upstream M.1
Glutamate
GltX
Glutamyl-tRNA
HemA
GSA
HemL
ALA
HemB
PBG
HemC
HMB
HemD
Uroporphyrinogen III
Downstream M.2
HemE
Coproporphyrinogen III
HemF
Protoporphyrinogen IX
HemG
Protoporphyrin IX
HemH
Heme
Downstream M.3
Cytochrome
Vitamin $B_{12}$
Coenzyme $F_{430}$
$^{15}N$_5-ALA
$^{15}N$ Protoporphyrin IX
NORMAL CELL
$Fe^{2+}$
(FECH)
$^{15}N$ Heme
(HMOX)
$Fe^{3+}$
$^{15}N$_ Biliverdin
$^{15}N$_ Bilirubin
EXCRETION OF $^{15}N$

Fig. 2

CENTER-OF-MASS SYSTEM ENERGY DIAGRAM OF PROTON CAPTURE RESONANCE NUCLEAR REACTION OF NITROGEN-15 ATOMIC NUCLEUS (UNIT = MeV)

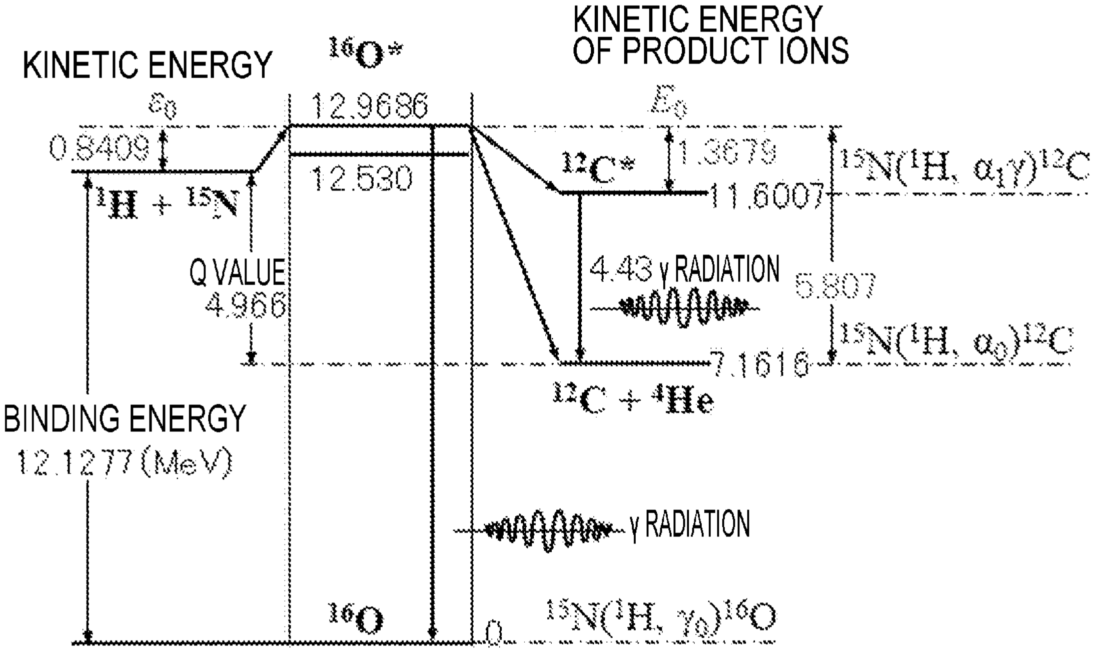


[$^1H$-$^{15}N$ CENTER-OF-MASS SYSTEM]

# Fig. 3

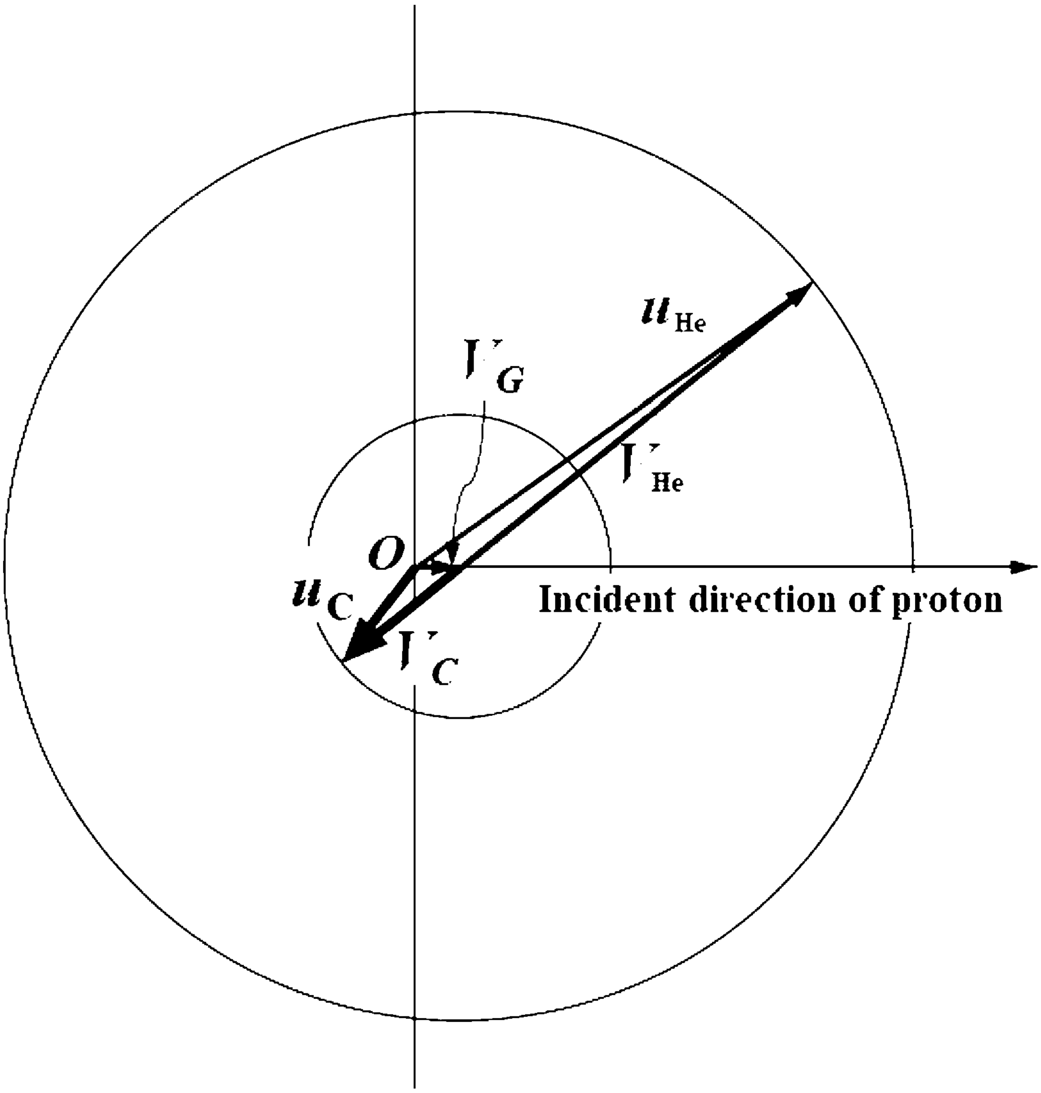

Fig. 4

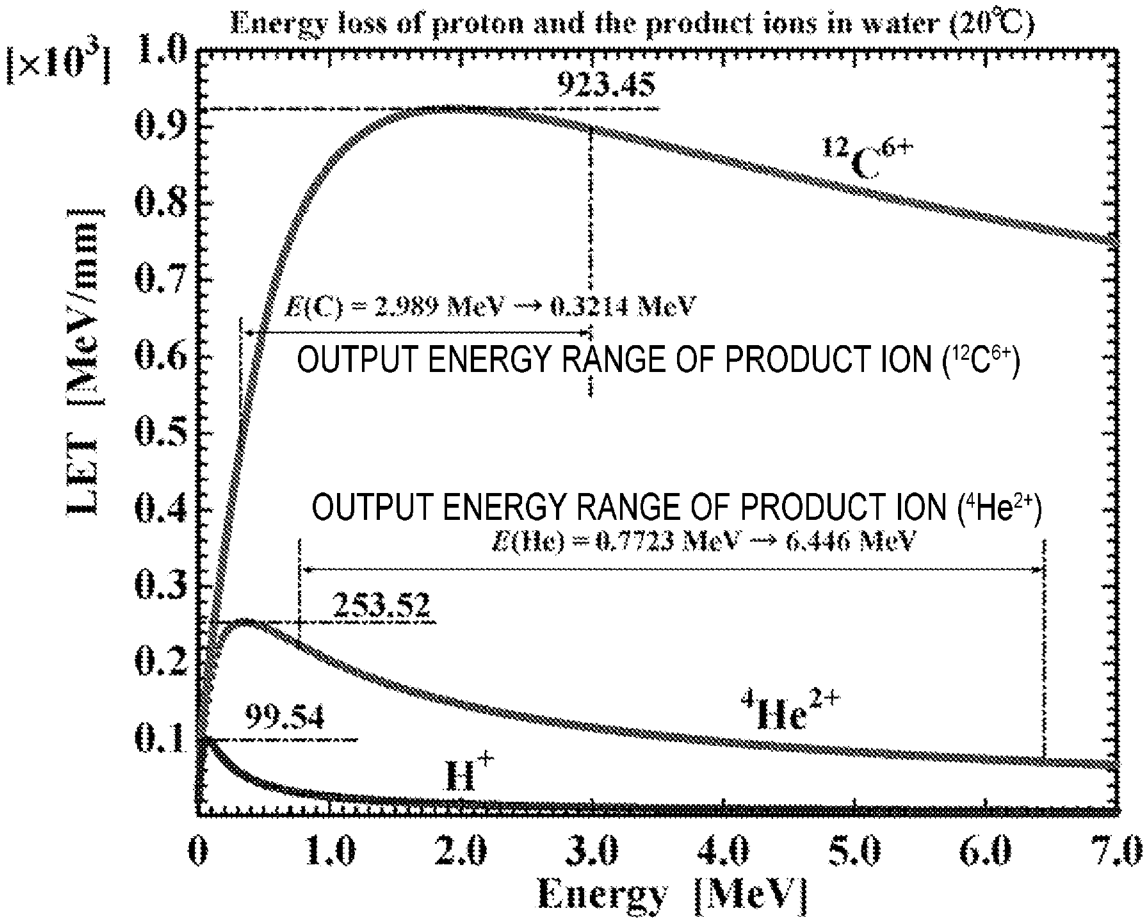
Energy loss of proton and the product ions in water (20℃)
[×10³]
LET [MeV/mm]
1.0
0.9
0.8
0.7
0.6
0.5
0.4
0.3
0.2
0.1
923.45
12C6+
E(C) = 2.989 MeV → 0.3214 MeV
OUTPUT ENERGY RANGE OF PRODUCT ION (12C6+)
OUTPUT ENERGY RANGE OF PRODUCT ION (4He2+)
E(He) = 0.7723 MeV → 6.446 MeV
253.52
4He2+
99.54
H+
0
1.0
2.0
3.0
4.0
5.0
6.0
7.0
Energy [MeV]

Fig. 5

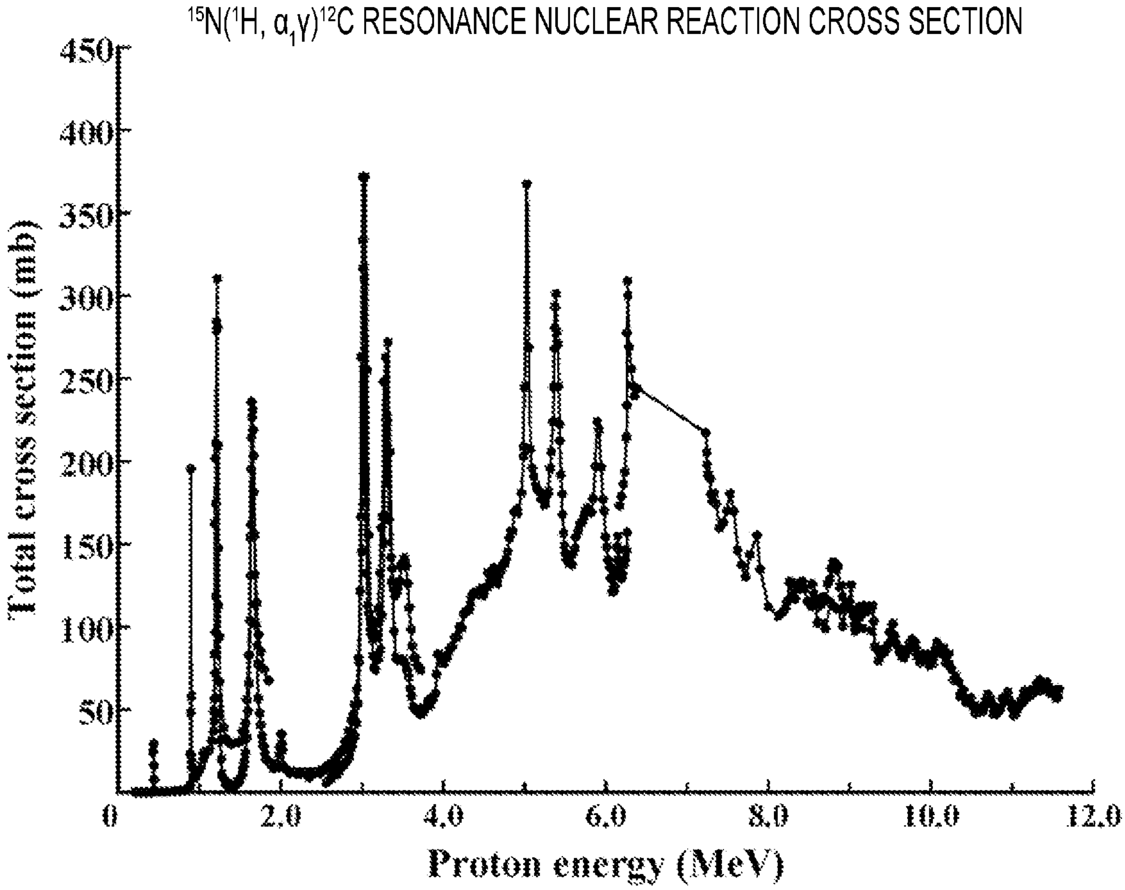
$^{15}N(^{1}H, \alpha_1\gamma)^{12}C$ RESONANCE NUCLEAR REACTION CROSS SECTION
Total cross section (mb)
450
400
350
300
250
200
150
100
50
0
2.0
4.0
6.0
8.0
10.0
12.0
Proton energy (MeV)

Fig. 6

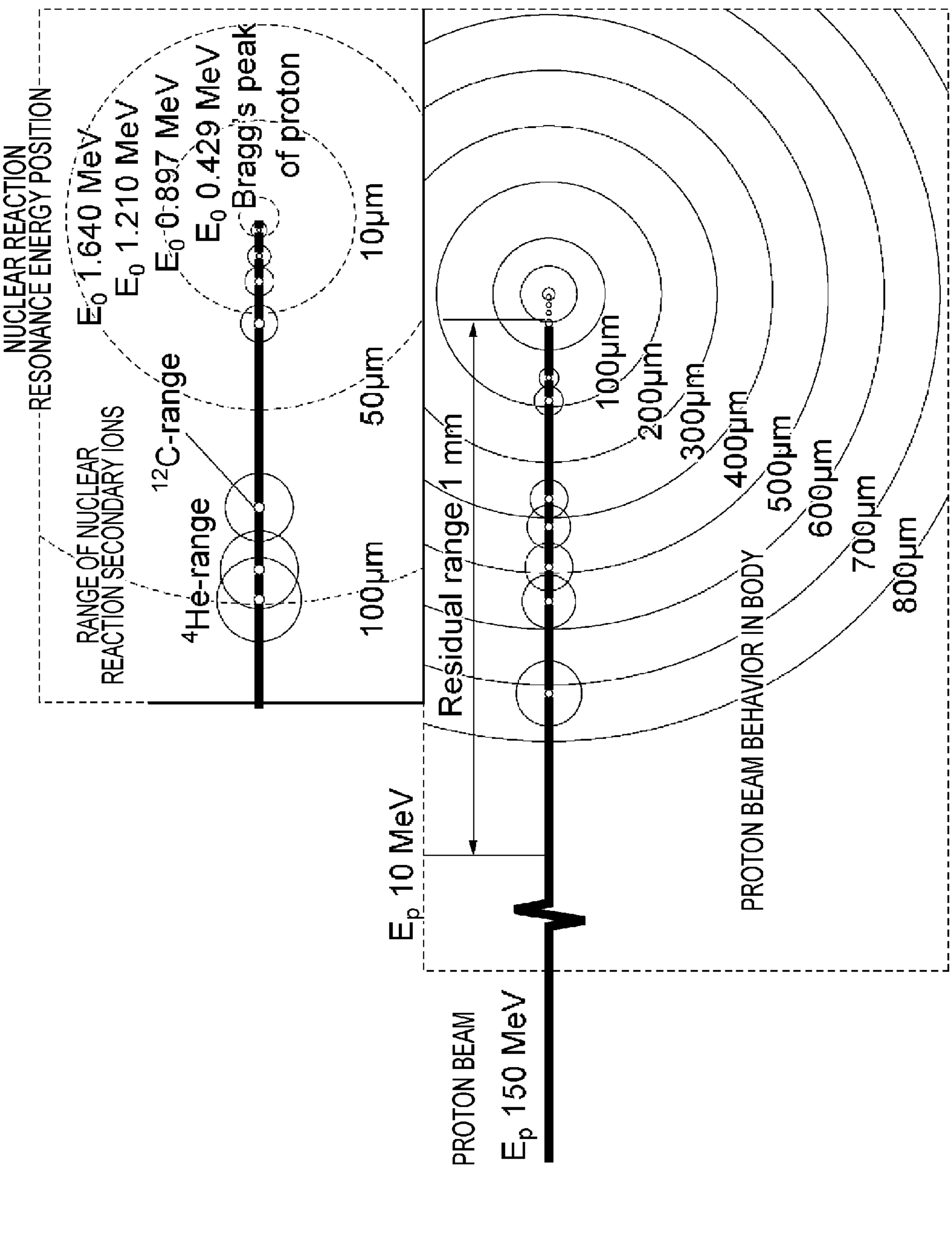
NUCLEAR REACTION RESONANCE ENERGY POSITION
RANGE OF NUCLEAR REACTION SECONDARY IONS
$E_0$ 1.640 MeV
$E_0$ 1.210 MeV
$E_0$ 0.897 MeV
$E_0$ 0.429 MeV
Bragg's peak of proton
$^{12}$C-range
$^4$He-range
10μm
50μm
100μm
Residual range 1 mm
100μm
200μm
300μm
400μm
500μm
600μm
700μm
800μm
PROTON BEAM BEHAVIOR IN BODY
$E_p$ 10 MeV
PROTON BEAM
$E_p$ 150 MeV

# Fig. 7

| Product ions | | $^4He^{2+}$ | | | $^{12}C^{6+}$ | | |
|---|---|---|---|---|---|---|---|
| Resonance energy (MeV) | Residual range (mm) | Kinetic energy (MeV) | Range (mm) | Bragg's peak ratio to proton (×times) | Kinetic energy (MeV) | Range (mm) | Bragg's peak ratio to proton (×times) |
| 0.42957 | 0.00645 | 0.7723 | 0.0038 | 2.55 | 0.3214 | 0.0013 | 4.85 |
| 0.89737 | 0.02010 | 1.1603 | 0.0057 | 2.55 | 0.5049 | 0.0016 | 6.47 |
| 1.21 | 0.03285 | 1.4189 | 0.0071 | 2.55 | 0.6267 | 0.0018 | 7.24 |
| 1.64 | 0.05455 | 1.7729 | 0.0093 | 2.55 | 0.7933 | 0.0020 | 7.96 |
| 2.982 | 0.15020 | 2.8786 | 0.0175 | 2.55 | 1.3131 | 0.0026 | 9.02 |
| 3.34 | 0.18240 | 3.1764 | 0.0201 | 2.55 | 1.4527 | 0.0028 | 9.14 |
| 3.499 | 0.19760 | 3.3050 | 0.0212 | 2.55 | 1.5134 | 0.0028 | 9.18 |
| 5.001 | 0.36675 | 4.5420 | 0.0338 | 2.55 | 2.0944 | 0.0034 | 9.28 |
| 5.375 | 0.41600 | 4.8503 | 0.0373 | 2.55 | 2.2392 | 0.0036 | 9.28 |
| 5.893 | 0.48875 | 5.2768 | 0.0425 | 2.55 | 2.4396 | 0.0038 | 9.28 |
| 6.297 | 0.54920 | 5.6092 | 0.0467 | 2.55 | 2.5957 | 0.0040 | 9.28 |
| 7.313 | 0.71505 | 6.4460 | 0.0579 | 2.55 | 2.9887 | 0.0044 | 9.28 |

Fig. 8

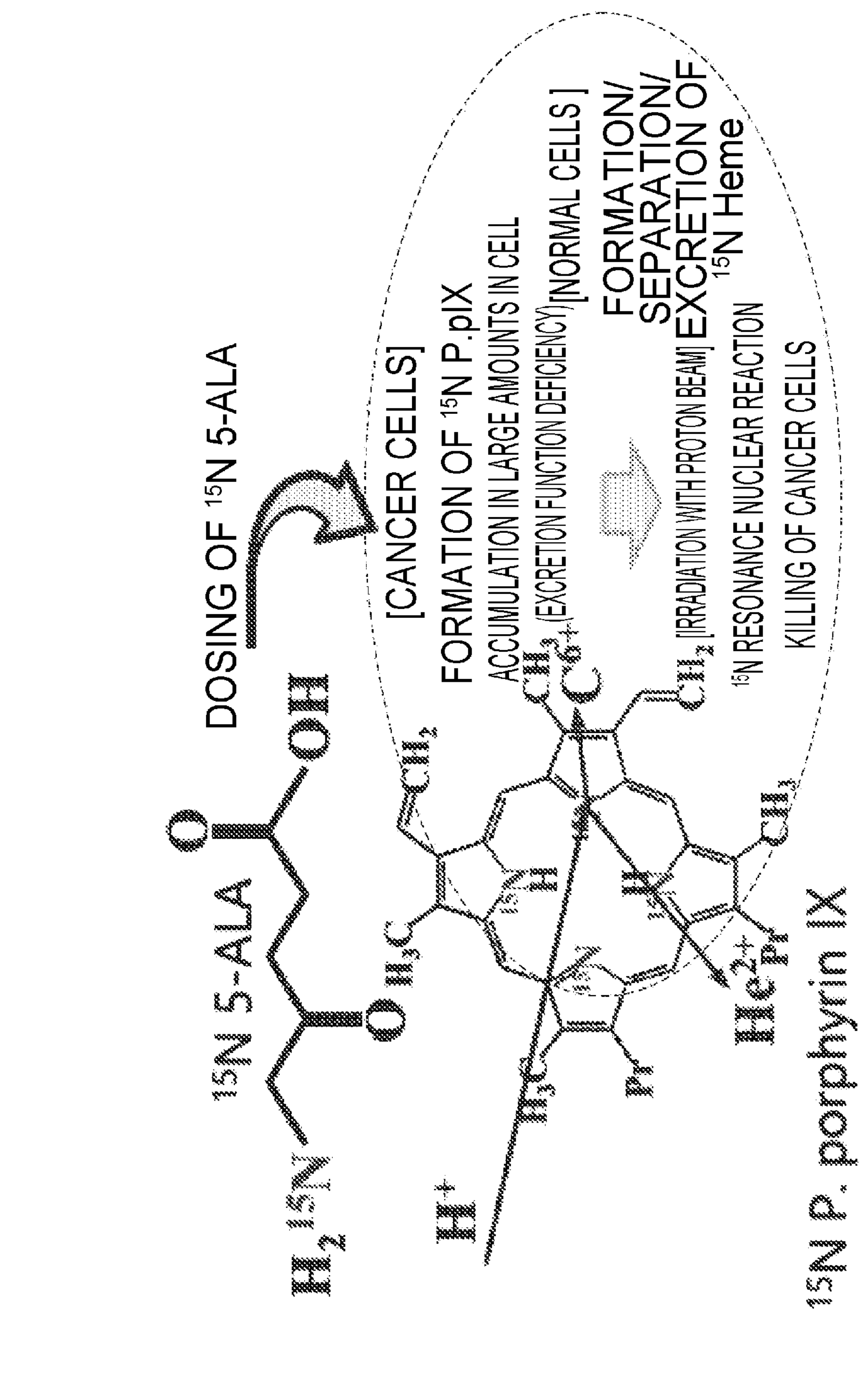
$^{15}$N 5-ALA
DOSING OF $^{15}$N 5-ALA
[CANCER CELLS]
FORMATION OF $^{15}$N P.pIX
ACCUMULATION IN LARGE AMOUNTS IN CELL
(EXCRETION FUNCTION DEFICIENCY)
[NORMAL CELLS]
FORMATION/
SEPARATION/
EXCRETION OF
$^{15}$N Heme
[IRRADIATION WITH PROTON BEAM]
$^{15}$N RESONANCE NUCLEAR REACTION
KILLING OF CANCER CELLS
$^{15}$N P. porphyrin IX

Fig. 9

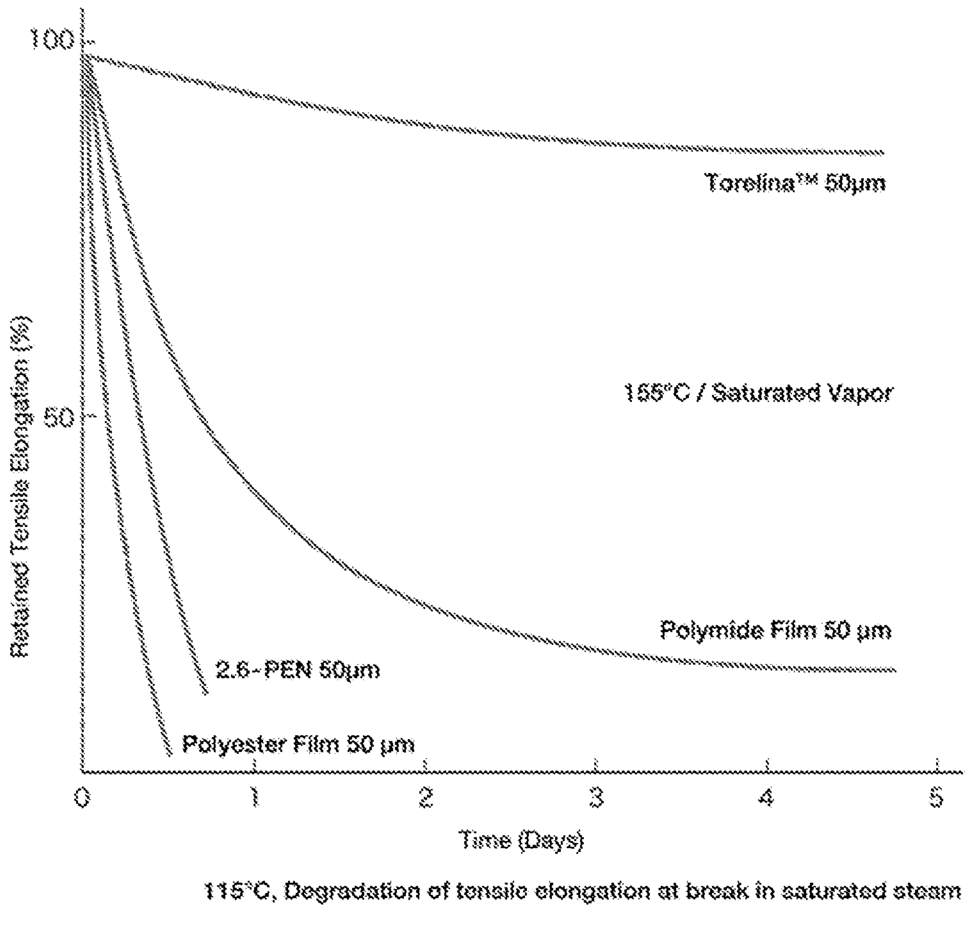


115°C, Degradation of tensile elongation at break in saturated steam

# Fig. 10

GAS PERMEABILITIES OF "TORELINA" AND POLYESTER FILM

| GAS | UNIT | "TORELINA" 25μm | "LUMIRROR" S10 POLYESTER FILM 25μm |
|---|---|---|---|
| OXYGEN ($O_2$) | mℓ・mm/(m²・day・MPa) | 50 | 19 |
| NITROGEN ($N_2$) | mℓ・mm/(m²・day・MPa) | 8.1 | 2.4 |
| HELIUM (He) | mℓ・mm/(m²・day・MPa) | 644 | —— |
| CARBON DIOXIDE ($CO_2$) | mℓ・mm/(m²・day・MPa) | 230 | 60 |
| WATER VAPOR ($H_2O$) | g・mm/(m²・day) | 0.25 | 0.55 |

Fig. 11

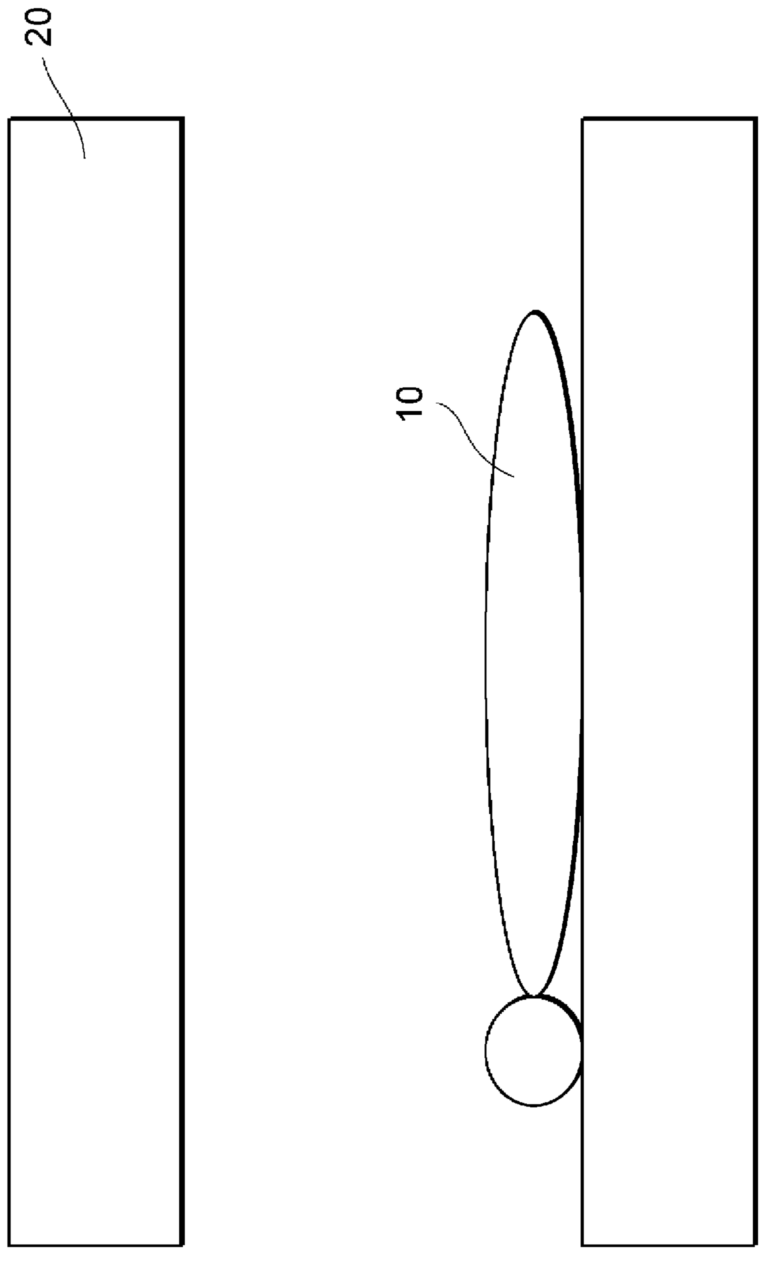

# Fig. 12

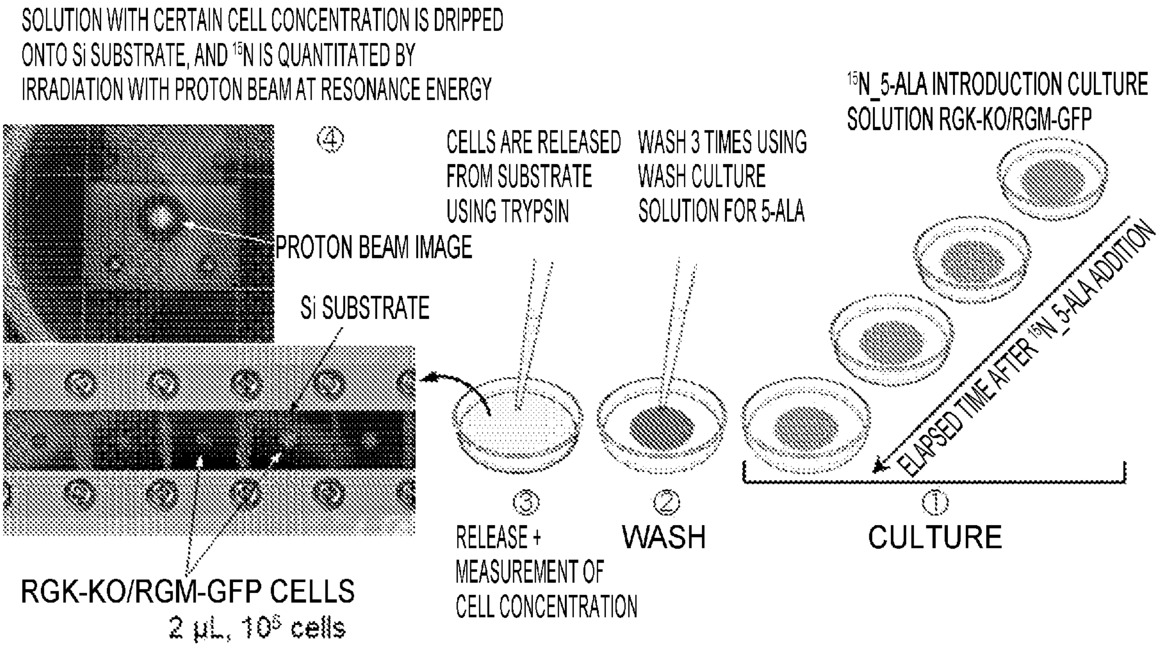
SOLUTION WITH CERTAIN CELL CONCENTRATION IS DRIPPED ONTO Si SUBSTRATE, AND 15N IS QUANTITATED BY IRRADIATION WITH PROTON BEAM AT RESONANCE ENERGY
④
PROTON BEAM IMAGE
Si SUBSTRATE
RGK-KO/RGM-GFP CELLS
2 μL, 10⁵ cells
CELLS ARE RELEASED FROM SUBSTRATE USING TRYPSIN
WASH 3 TIMES USING WASH CULTURE SOLUTION FOR 5-ALA
15N_5-ALA INTRODUCTION CULTURE SOLUTION RGK-KO/RGM-GFP
ELAPSED TIME AFTER 15N_5-ALA ADDITION
③
RELEASE + MEASUREMENT OF CELL CONCENTRATION
②
WASH
①
CULTURE

Fig. 13

GAMMA RAY SPECTRUM

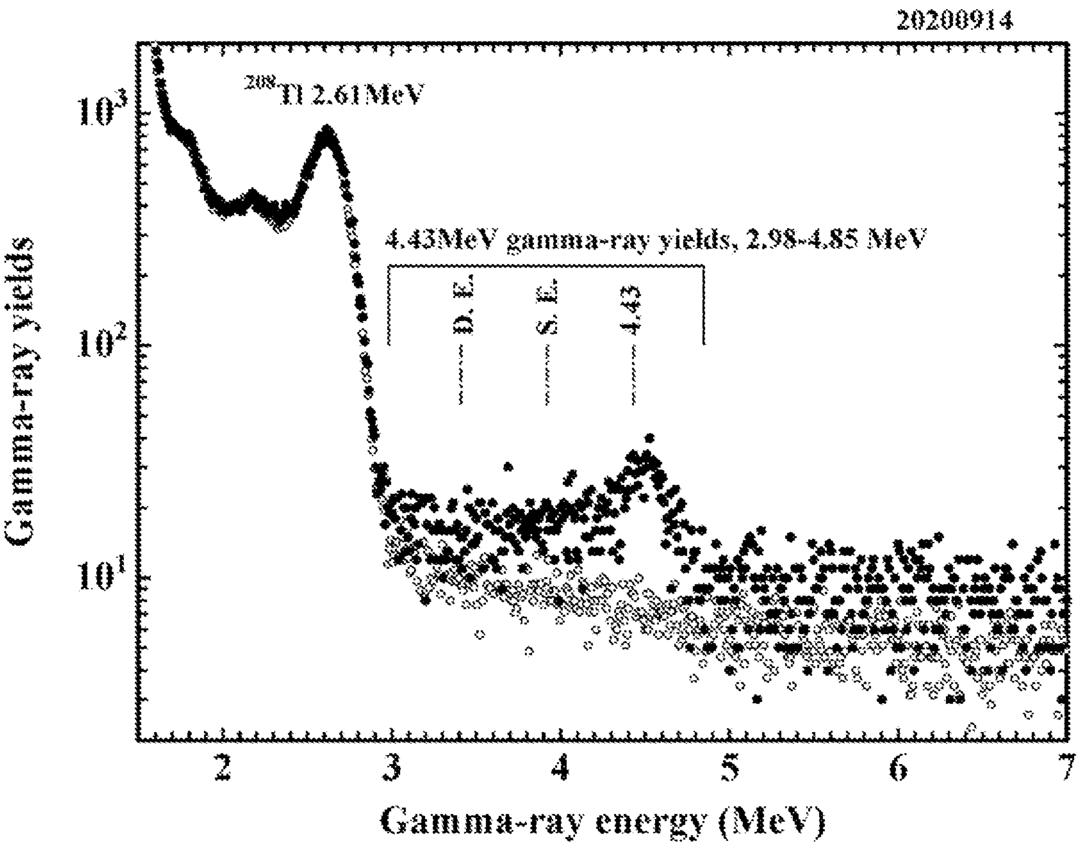

# Fig. 14

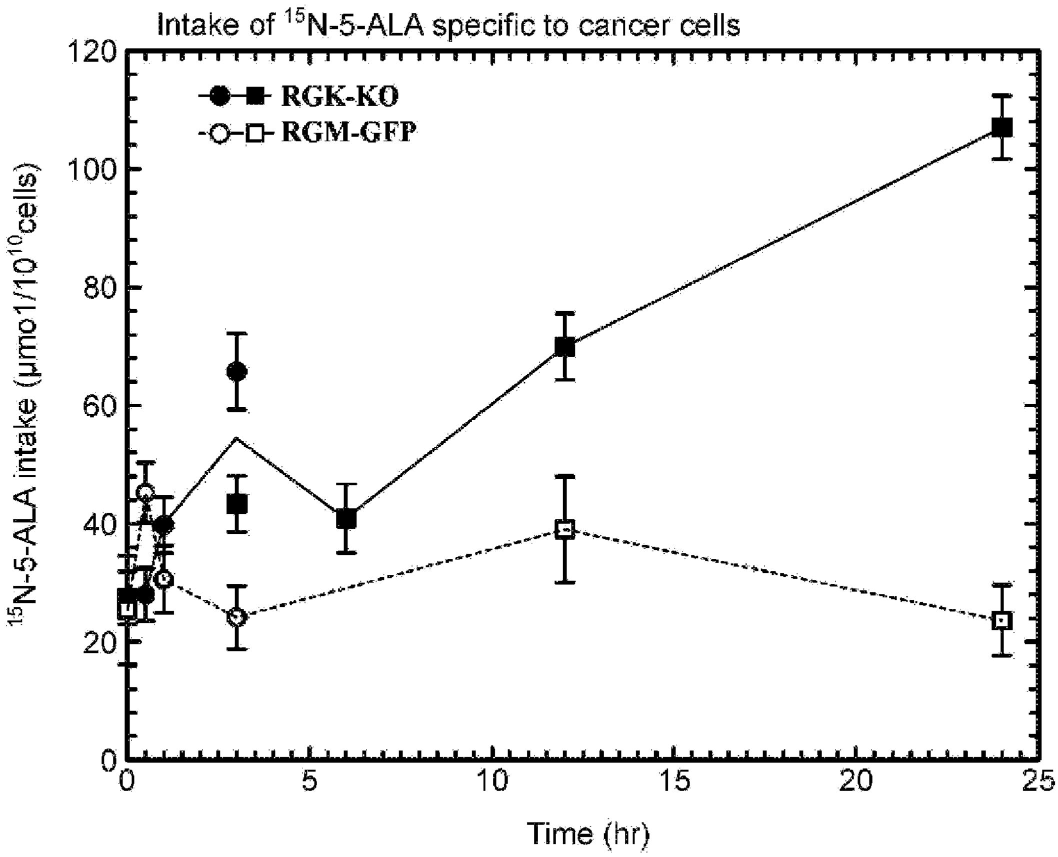
Intake of ¹⁵N-5-ALA specific to cancer cells
RGK-KO
RGM-GFP
¹⁵N-5-ALA intake (μmo1/10¹⁰cells)
Time (hr)
0
20
40
60
80
100
120
0
5
10
15
20
25

Fig. 15

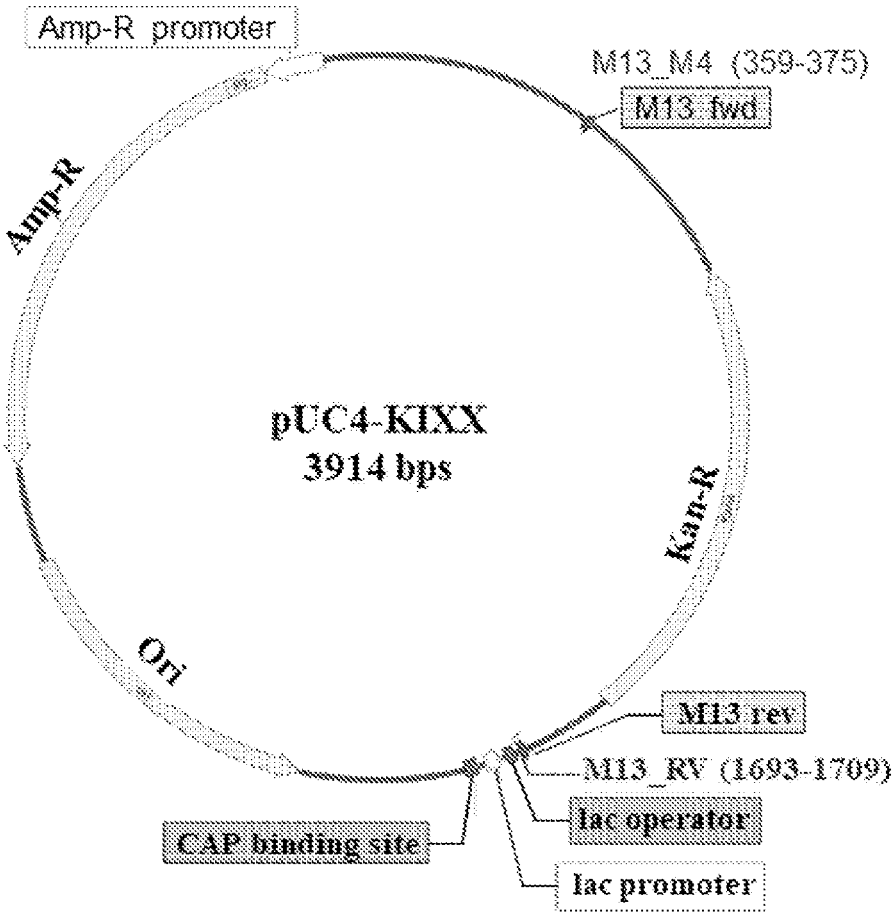
Amp-R promoter
M13_M4 (359-375)
M13 fwd
Amp-R
pUC4-KIXX
3914 bps
Kan-R
Ori
M13 rev
M13_RV (1693-1709)
lac operator
CAP binding site
lac promoter

Fig. 16

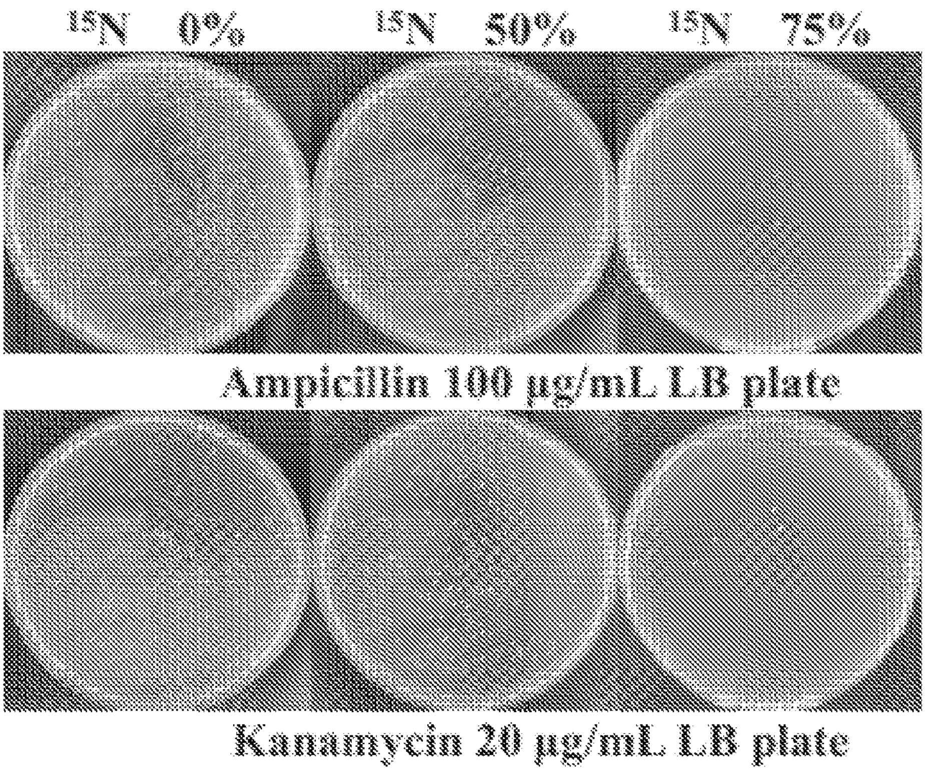
$^{15}N$ 0%
$^{15}N$ 50%
$^{15}N$ 75%
Ampicillin 100 µg/mL LB plate
Kanamycin 20 µg/mL LB plate

# Fig. 17

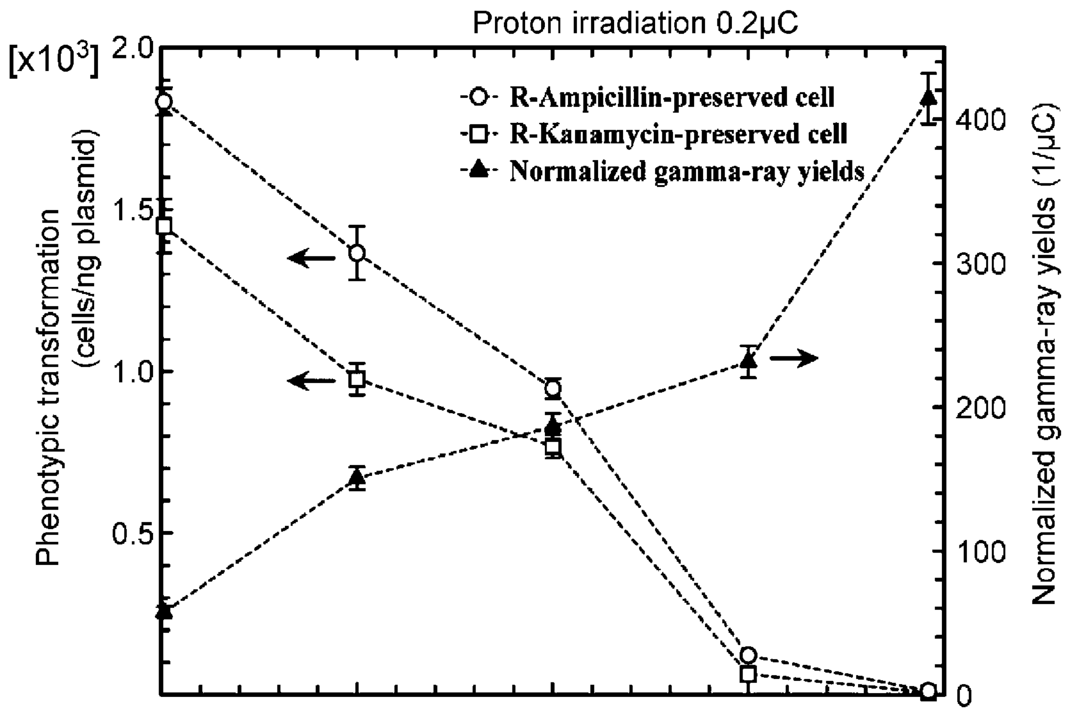
Proton irradiation 0.2μC
[x10³]
2.0
1.5
1.0
0.5
Phenotypic transformation
(cells/ng plasmid)
-O- R-Ampicillin-preserved cell
-□- R-Kanamycin-preserved cell
-▲- Normalized gamma-ray yields
400
300
200
100
0
Normalized gamma-ray yields (1/μC)
0
25
50
75
100
¹⁵N-isotope abundance (%)

# Fig. 18

PHOTOGRAPHS (1) OF PROTON BEAM-IRRADIATED CELLS, RGM-GFP

RGM-GFP RGM-GFP RGM-GFP

PROTON BEAM IRRADIATION CHARGE AMOUNT 2.5 nC 4.0 nC 5.0 nC

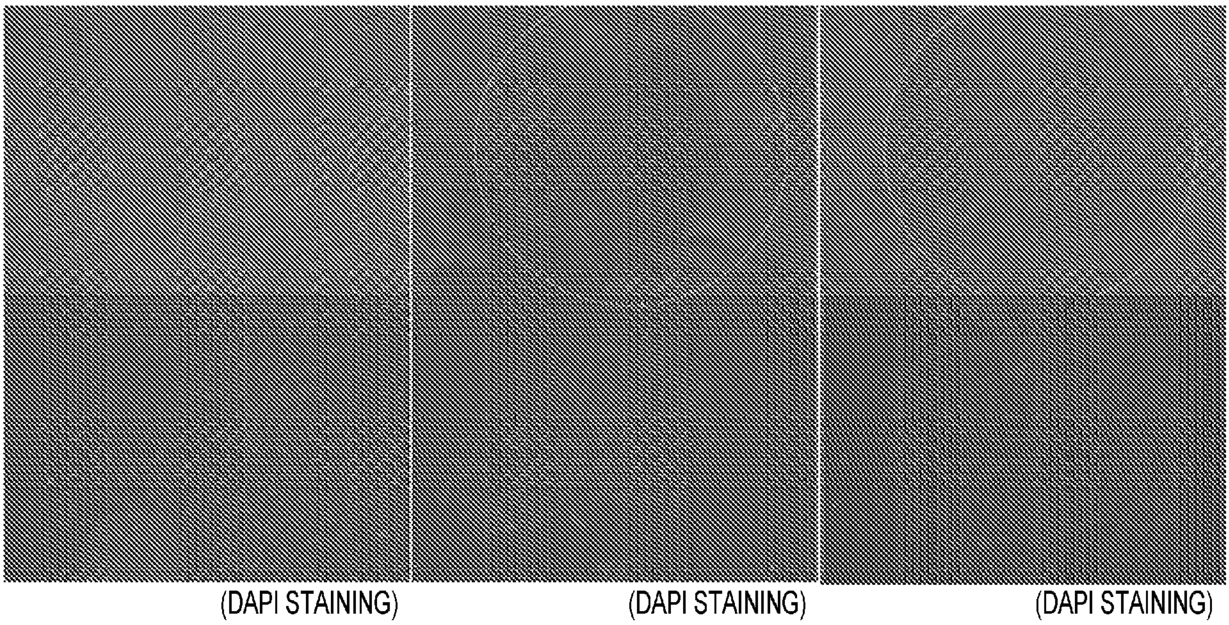

(DAPI STAINING) (DAPI STAINING) (DAPI STAINING)

SURVIVING FRACTION 78.7% SURVIVING FRACTION 44.0% SURVIVING FRACTION 11.1%

# Fig. 19

PROTON BEAM IRRADIATION DOSE AND
CELL SURVIVING FRACTION, RGM-GFP GRAPH

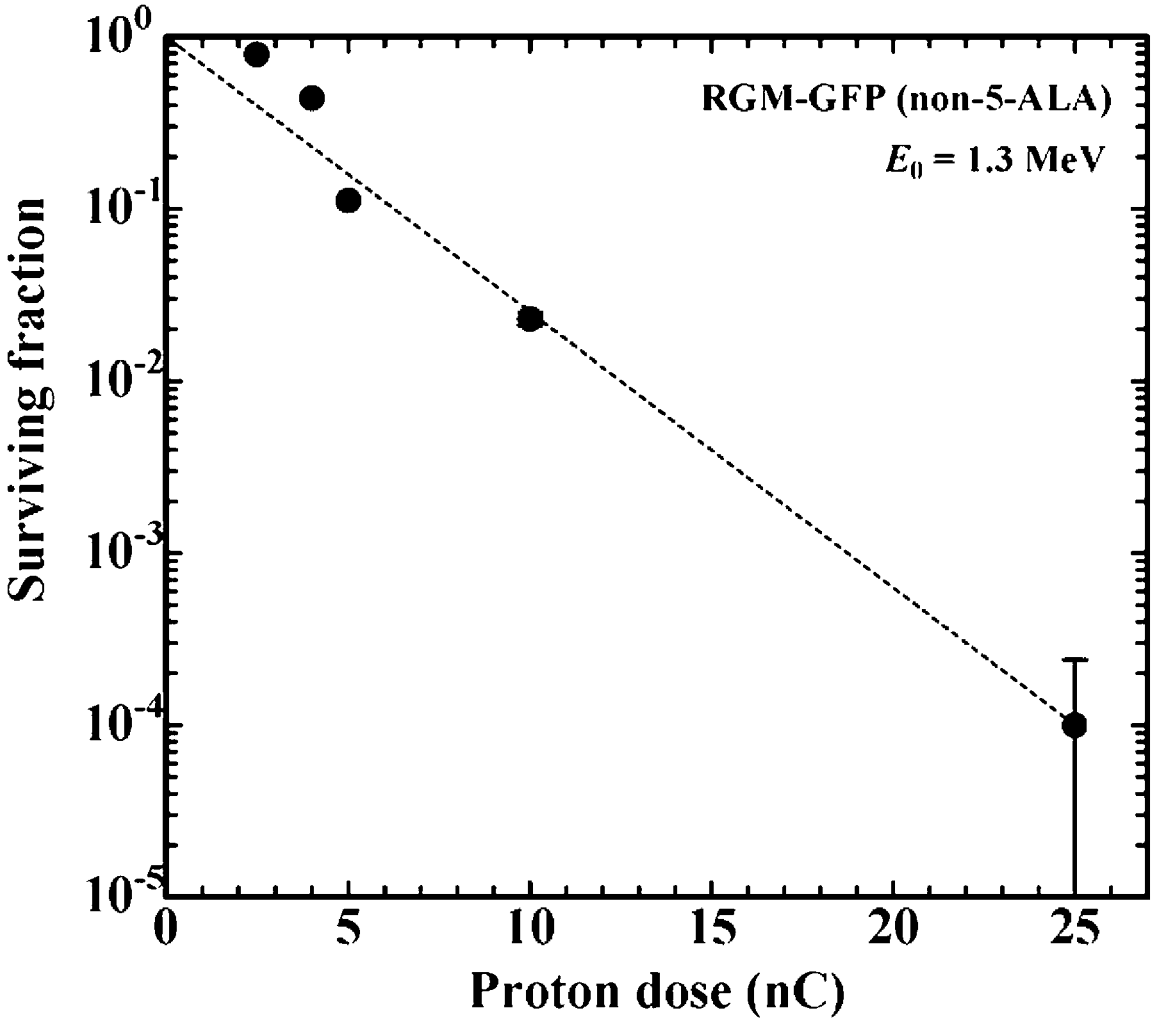

# Fig. 20

PHOTOGRAPHS (2) OF PROTON BEAM-IRRADIATED CELLS,
RGK-KO IMMEDIATELY AFTER IRRADIATION

RGK-KO (NO ALA) | RGK-KO ($^{15}N_5$-ALA)

PROTON BEAM IRRADIATION CHARGE AMOUNT 4.0 nC | PROTON BEAM IRRADIATION CHARGE AMOUNT 4.0 nC

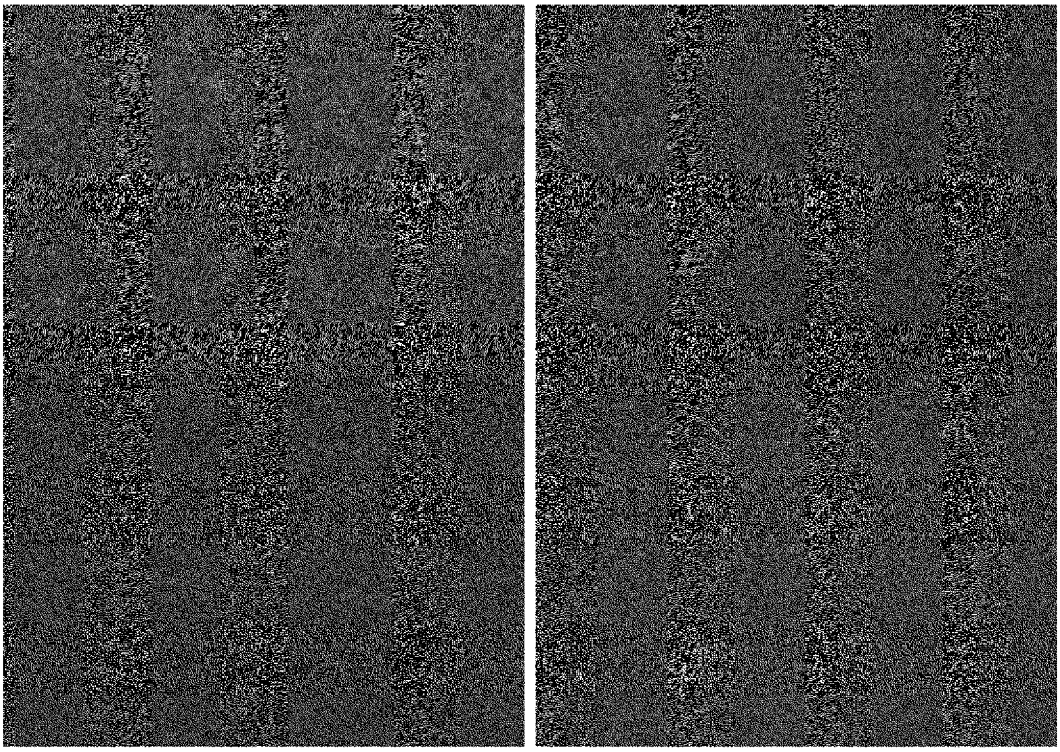

(DAPI STAINING) | (DAPI STAINING)

# Fig. 21

PHOTOGRAPHS (3) OF PROTON BEAM-IRRADIATED CELLS, RGK-KO,
3 HOURS AFTER IRRADIATION/24 HOURS ELAPSED AFTER IRRADIATION

| RGK-KO(15N-ALA) PROTON BEAM IRRADIATION CHARGE AMOUNT 4.0 nC | | RGK-KO(15N-ALA) PROTON BEAM IRRADIATION CHARGE AMOUNT 4.0 nC | |
|---|---|---|---|
| 3 HOURS AFTER IRRADIATION | 24 HOURS AFTER IRRADIATION | 3 HOURS AFTER IRRADIATION | 24 HOURS AFTER IRRADIATION |

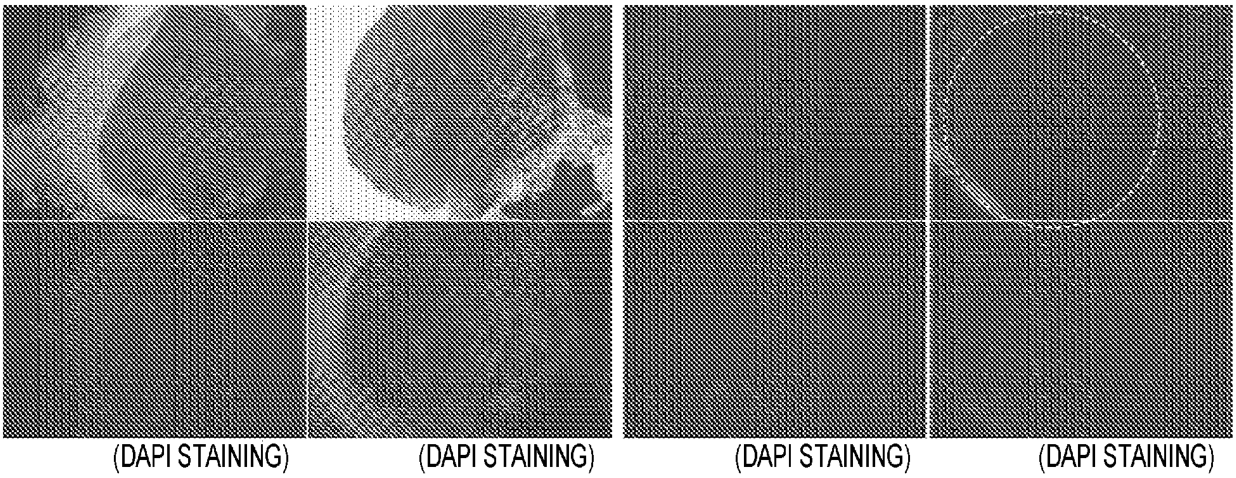

| (DAPI STAINING) | (DAPI STAINING) | (DAPI STAINING) | (DAPI STAINING) |
|---|---|---|---|
| SURVIVING FRACTION 85.5% | SURVIVING FRACTION 10.6% | SURVIVING FRACTION 73.5% | SURVIVING FRACTION 40.5% |

Fig. 22

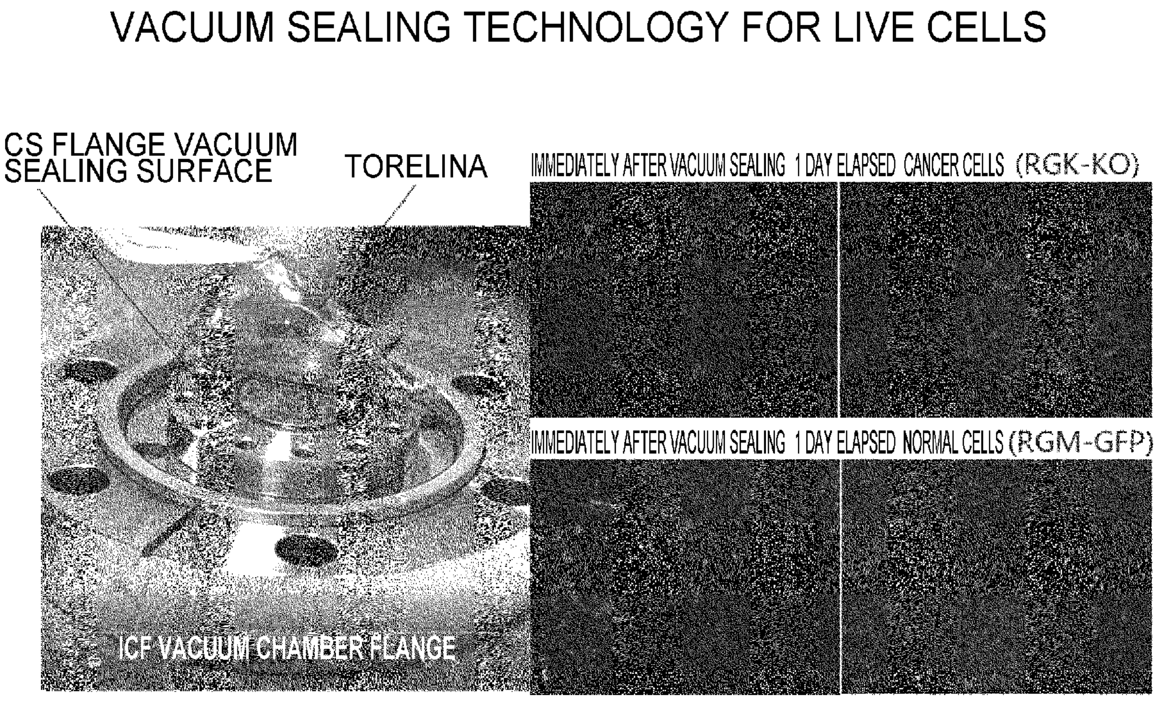
VACUUM SEALING TECHNOLOGY FOR LIVE CELLS
CS FLANGE VACUUM SEALING SURFACE
TORELINA
ICF VACUUM CHAMBER FLANGE
IMMEDIATELY AFTER VACUUM SEALING 1 DAY ELAPSED CANCER CELLS (RGK-KO)
IMMEDIATELY AFTER VACUUM SEALING 1 DAY ELAPSED NORMAL CELLS (RGM-GFP)

# Fig. 23

SEALING TECHNOLOGY-VACUUM (GRAPH)

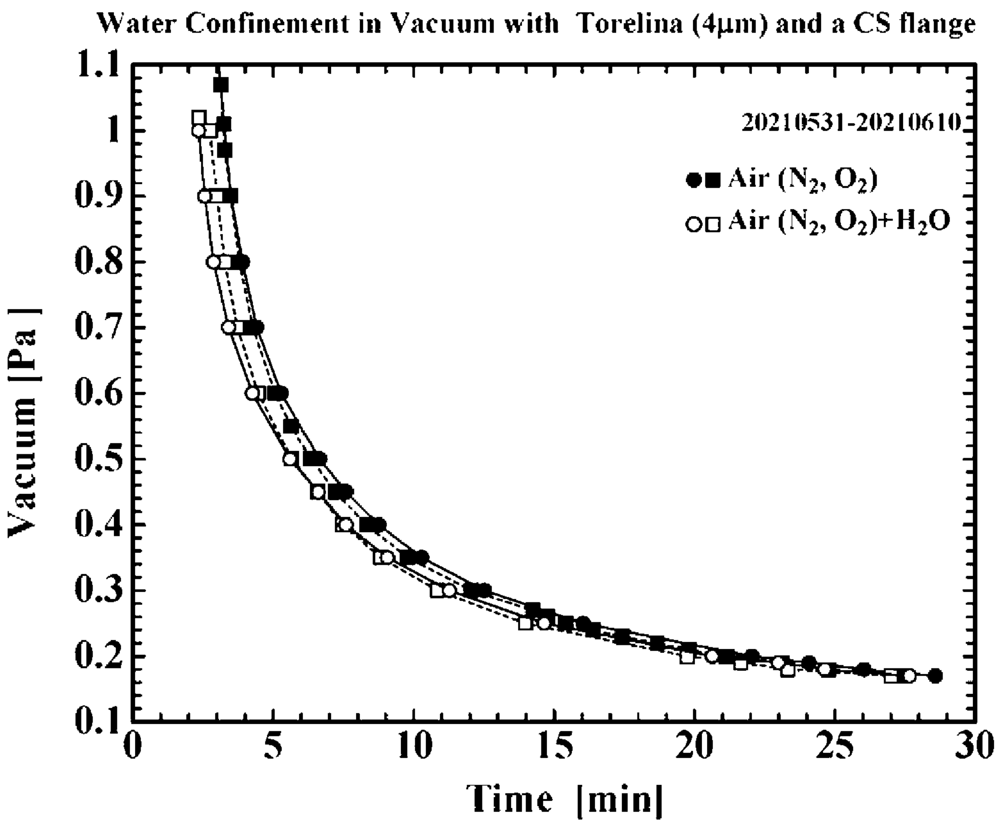

## INTERNATIONAL SEARCH REPORT

International application No.
**PCT/JP2022/039079**

**A. CLASSIFICATION OF SUBJECT MATTER**

***A61K 41/00***(2020.01)i; ***A61K 31/197***(2006.01)i; ***A61K 31/513***(2006.01)i; ***A61K 45/00***(2006.01)i; ***A61K 47/68***(2017.01)i; ***A61N 5/10***(2006.01)i; ***A61P 35/00***(2006.01)i; ***C12N 5/09***(2010.01)i
FI: A61K41/00; A61K31/197; A61K31/513; A61K45/00; A61K47/68; A61N5/10 Z; A61P35/00; C12N5/09

According to International Patent Classification (IPC) or to both national classification and IPC

**B. FIELDS SEARCHED**

Minimum documentation searched (classification system followed by classification symbols)

A61K41/00; A61K31/197; A61K31/513; A61K45/00; A61K47/68; A61N5/10; A61P35/00; C12N5/09

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 11-012197 A (COSMO SOGO KENKYUSHO KABUSHIKI KAISHA) 19 January 1999 (1999-01-19)<br>claim 2, production examples 1, 2, paragraph [0032] | 1, 3, 10 |
| X | CN 112834680 A (HQ BIOSCIENCE CO., LTD.) 25 May 2021 (2021-05-25)<br>fig. 5, 6 | 1, 4, 10, 12, 13 |
| Y | JP 2018-527402 A (SYNTHON BIOPHARMACEUTICALS B. V.) 20 September 2018 (2018-09-20)<br>claim 1 | 1, 5-9 |
| Y | JP 2018-58822 A (PFIZER INC.) 12 April 2018 (2018-04-12)<br>claim 11 | 1, 5-9 |
| Y | JP 2003-096098 A (NIPPON OXYGEN CO., LTD.) 03 April 2003 (2003-04-03)<br>claim 1, paragraph [0009] | 1, 5-9 |
| X | JP 2008-022994 A (JAPAN ATOMIC ENERGY AGENCY) 07 February 2008 (2008-02-07)<br>claim 11, paragraph [0001] | 20-22 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 January 2023** | **17 January 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/039079**

**C. DOCUMENTS CONSIDERED TO BE RELEVANT**

| Category* | Citation of document, with indication, where appropriate, of the relevant passages | Relevant to claim No. |
|---|---|---|
| A | JP 2016-216404 A (NATIONAL UNIVERSITY CORPORATION HOKKAIDO UNIVERSITY) 22 December 2016 (2016-12-22) claim 1 | 1-22 |
| A | JP 2014-177421 A (FUKUI UNIVERSITY) 25 September 2014 (2014-09-25) claim 1 | 1-22 |
| A | 岩田康嗣, 医療機関、企業、大学、行政の連携が創る独自医療技術開発, 日レ医誌, 30 September 2021, vol. 42, no. 3, p. 138 non-official translation (IWATA, Yasushi. Developing unique medical technology to link medical institutions, companies, universities and government offices. JSLSM Journal.) entire text | 1-22 |
| A | WO 2021/029391 A1 (THE NATIONAL INSTITUTE OF ADVANCED INDUSTRIAL SCIENCE AND TECHNOLOGY) 18 February 2021 (2021-02-18) claim 10 | 1-22 |
| A | WO 2018/237241 A1 (UNIVERSITY OF VIRGINIA PATENT FOUNDATION) 27 December 2018 (2018-12-27) claim 10 | 1-22 |

Form PCT/ISA/210 (second sheet) (January 2015)

**INTERNATIONAL SEARCH REPORT**
**Information on patent family members**

International application No.
**PCT/JP2022/039079**

| Patent document cited in search report | Publication date (day/month/year) | Patent family member(s) | Publication date (day/month/year) |
|---|---|---|---|
| JP 11-012197 A | 19 January 1999 | US 2003/0065029 A1<br>claims 2, 4, production examples 1, 2, paragraph [0045]<br>WO 1998/057668 A1<br>EP 995448 A1 | |
| CN 112834680 A | 25 May 2021 | (Family: none) | |
| JP 2018-527402 A | 20 September 2018 | US 2018/0280533 A1<br>claim 1<br>WO 2017/050846 A1<br>EP 3352857 A1<br>CN 108025190 A<br>KR 10-2018-0052761 A | |
| JP 2018-58822 A | 12 April 2018 | US 2021/0308276 A1<br>claim 11<br>WO 2018/025168 A1<br>EP 3493853 A1 | |
| JP 2003-096098 A | 03 April 2003 | (Family: none) | |
| JP 2008-022994 A | 07 February 2008 | (Family: none) | |
| JP 2016-216404 A | 22 December 2016 | (Family: none) | |
| JP 2014-177421 A | 25 September 2014 | (Family: none) | |
| WO 2021/029391 A1 | 18 February 2021 | US 2022/0298499 A1<br>claim 10<br>EP 4042862 A1<br>CN 114207127 A | |
| WO 2018/237241 A1 | 27 December 2018 | US 2021/0024564 A1<br>EP 3641783 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4520219 B
- JP 7648586 B
- JP 5392752 B


### Non-patent literature cited in the description


- **F. TABBAKH ; N. S. HOSMANE.** Enhancement of Radiation Effectiveness in Proton Therapy: Comparison Between Fusion and Fission Methods and Further Approaches. *Scientific Reports,* 2020, vol. 10, 5466
- **TORU TANAKA et al.** Production of ByProducts During 5-Aminolevulinic Acid Production by a Mutant Photosynthetic Bacteria and Industrial Production of 5-Aminolevulinic Acid Under Temperature Control. *Seibutsu Kogaku Kaishi,* 2015, vol. 93, 24-31
- **C. ROLFS ; W. S. RODNEY.** PROTON CAPTURE BY 15N AT STELLAR ENERGIES. *Nuclear Physics,* 1974, vol. A235, 450-459
- **G. IMBRIANI et al.** Measurement of $\gamma$ rays from 15N(p,$\gamma$)16O cascade and 15N(p,$\alpha$1$\gamma$)12C reactions. *PHYSICAL REVIEW,* 2012, vol. C85, 065810
- Torelina (registered trademark) Polyphenylene Sulfide Film. Technical Data. Industrial Materials First Business Unit, Toray Industries, Inc, 2013
- **M. TAMURA ; H. ITO ; H. MATSUI.** Radiotherapy for cancer using X-ray fluorescence emitted from iodine. *Scientific Reports,* 2017, vol. 7, 43667
- **M. SHIBUTA et al.** Imaging cell picker: A morphology-based automated cell separation system on a photodegradable hydrogel culture platform. *J. Bioscience and Bioengineering,* 2018, vol. 126, 653-660